# EUROPEAN PATENT SPECIFICATION

(11) **EP 4 303 313 B1**
(45) Date of publication and mention of the grant of the patent: **11.03.2026**
(21) Application number: 22879597.7
(22) Date of filing: 11.08.2022
(51) Int. Cl.: C12Q 1/6806, C12Q 1/6811

(54) **LIQUID-PHASE HYBRID CAPTURE METHOD AND TEST KIT THEREOF**
HYBRIDES FLÜSSIGPHASENERFASSUNGSVERFAHREN UND HYBRIDES FLÜSSIGPHASENERFASSUNGSKIT
PROCÉDÉ DE CAPTURE HYBRIDE EN PHASE LIQUIDE ET KIT DE TEST ASSOCIÉ

(30) Priority: 16.05.2022 CN 202210531282
(43) Date of publication of application: 10.01.2024
(73) Proprietor: Nanodigmbio (Nanjing) Biotechnology Co., Ltd, Jiangsu 210000 (CN)
(72) Inventor: YU, Liping, Nanjing, Jiangsu 210000 (CN); WANG, Biao, Nanjing, Jiangsu 210000 (CN); JI, Changquan, Nanjing, Jiangsu 210000 (CN); WU, Qiang, Nanjing, Jiangsu 210000 (CN)
(74) Representative: Huang, Liwei
(86) International application number: PCT/CN2022/111685
(87) International publication number: WO 2023/221308

(56) References cited:
- EP-A1- 3 730 613
- WO-A1-2018/057999
- WO-A1-2023/221307
- CN-A- 110 352 252
- CN-A- 114 891 859
- JP-A- 2005 304 489
- US-A1- 2007 184 436
- US-A1- 2017 175 177
- US-A1- 2021 040 540

## Description

### Field

The present invention relates to the field of genetic detection, in particular to a liquid-phase hybrid capture method.

### Background

A nucleic acid sequence is a carrier of life information, while a high-throughput sequencing technology has become one of the core technologies in the biological and medical fields. High-throughput sequencing produces a large amount of data, not all of which are target sequences for research or detection. Although the cost of sequencing has been significantly reduced, due to the high volume of whole genome sequencing data, the cost is still high, and a solution to this problem is to change whole genome sequencing into a targeted enrichment technique. A target region-enriched NGS sequencing technique will ignore information from regions of non-interest in a genome and amplify signals from a target region in the genome, which can save the sequencing cost and the sequencing time.

Targeted enrichment is mainly divided into multiplex PCR amplification and targeted capture based on different enrichment principles. The latter is a probe-based liquid-phase hybrid capture technology, is a mainstream at present, and has the advantages of low probe design difficulty and high probe fault tolerance. The liquid-phase hybrid capture technology is that a biotin-labeled probe specifically binds to a target region in a solution, and target fragments captured by the probe are enriched by streptavidin magnetic beads. During this process, the probe labeled with biotin and liquid phase reaction conditions of hybrid capture have a significant impact on the capture efficiency of this system. For a large target region, the hybrid capture efficiency is higher, for example, a whole exon target region (Panel, also known as a capture region) has an on-target rate of 80% or more; however, for some small target regions (Panels), the on-target rate is relatively low, for example, a small target region of 10 kb or below has an on-target rate of a single digit or below.

The selection of a probe sequence length has various considerations: first, the probe length should ensure that in a specific hybridization system, under different sequence base compositions, the hybridization annealing temperature is appropriate, and the binding ability and specificity of the probe with a target sequence are optimal; secondly, it should be ensured that when there is a certain degree of mismatch between sequences of the probe and the target sequence, the hybridization annealing temperature does not decrease significantly; and finally, the longer the probe, the more difficult to synthesize it, and the more difficult to ensure the quality of synthesis. Currently, based on the above considerations, the probe sequence length is generally 40-120 nt, while the mainstream probe length is 120 nt, and is modified (such as biotin), and its modified group can bind to a corresponding affinity medium to complete the "capture" of the target sequence. The forms of the probe include single-stranded DNA, double-stranded DNA, single-stranded RNA, double-stranded RNA, and the like.

Currently, a second generation sequencing technology is the most widely used high-throughput sequencing technology, with bi-directional 150 bp being a more mainstream sequencing reading mode. The average insert fragment length of a sequencing library is also 100-400bp. The middle part of an excessively long insert fragment cannot be read, and the excessively long fragment also poses a challenge to multiple PCR amplification steps in the sequencing process. In addition, for samples with a short original length, such as FFPE and extracellular free nucleic acid, it is impossible to prepare a library with longer insert fragments. Then, one library molecule typically can only bind to 1-2 probes during hybrid capture, which also means that the probability of probe detachment increases and the recovery rate of the target sequence decreases. For example, a target sequence of 120 bp in length can only bind to one probe completely at most, and even if the target sequence can bind to two probes, the two probes can only be partially bound. In order to increase the binding capacity and probability of probes, the probes may be shortened, or an imbricated design strategy may be adopted, i.e., the probes are overlapped with each other so that different target sequence fragments have a higher probability of more complete binding to the probes. However, even probes which are overlapped with each other cannot completely bind to the same target fragment simultaneously.

For a sequencing library subjected to PCR amplification, there are multiple copies in each target fragment, and therefore a lower recovery rate can also ensure that most of the original target fragments have captured copies. And the hybrid capture technology typically targets regions of 5 kb or more, while for inherent non-specific capture, compression can be performed by a variety of means, with an on-target rate (a proportion of a target sequence in all captured sequences) being guaranteed to a certain extent. However, current mainstream probes and hybrid capture systems do not provide a satisfactory recovery efficiency and on-target rate for a sequencing library that has short insert fragments, or is not subjected to PCR amplification, and an application requirement with a low proportion of target regions in total regions.

In addition, the liquid-phase hybrid capture process is very time-consuming, taking 2-4 days from a nucleic acid sample to capture library obtaining; meanwhile, hybrid capture involves a large number of reagents, is an extremely cumbersome operation process, and has high technical requirements for operators. A problem in any link of the process will affect the performance of the capture library. These links become critical technical bottlenecks that restrict the development of liquid-phase hybrid capture.

The liquid-phase hybrid capture technology is widely used in cancer tumor mutation gene detection, copy number variation, and methylation status analysis. At present, many products are applied to gene detection and clinical application research in the market. However, with the rise in the popularity of early screening of tumors and MRD, higher requirements are put forward for the liquid-phase hybrid capture technology. For example, for a solid tumor MRD detection technology, primary tumor tissue is first sequenced to identify patient-specific genomic variation maps, and then a target region is designed for personalized ctDNA detection analysis. This requires higher requirements for a hybrid capture system in terms of compatibility with small target regions, ease of operation, degeneracy of experimental processes, and degree of automation.

Therefore, developing a probe with high recovery efficiency and a high on-target rate, as well as a liquid-phase hybrid capture system with high capture efficiency, uniformity, stability, and easy operation, fewer types of reagents, and short time consumption is a solution to solve the problems in the current market.

Some prior arts can be seen in WO2018/057999A1, WO2019/200580A1, EP3730613A1.

### Summary

An objective of the present invention is to provide a liquid-phase hybrid capture method directed against the limitations in the prior art such as a complex hybridization enrichment solution and a long reaction time.

The invention is set out in the appended set of claims.

The present disclosure also provides a design method for the pool of probes, including the following steps of:
(1) generating sequence information by inputting pre-capture library information and design parameters, wherein the sequence information includes total sequence information and target sequence information, and the design parameters include an annealing temperature range, and a sequence length range for binding of probes to a target sequence;
(2) counting the number of occurrences of all sequence combinations with a length of k in a plus strand and a complementary strand in the sum sequence, wherein k is less than the minimum value in the sequence length range of binding regions of the probes to target sequences;
(3) selecting a probe binding sequence in which probes are complementary paired, wherein the probe binding sequence has a length of k, and has an annealing temperature that is less than the annealing temperature for binding of the probes to the target sequence, and occurs less frequently in the sum sequence, preferably, the number of occurrences is less than 5% of the average;
(4) selecting a target specific sequence in which the probes bind to a nucleic acid target sequence, wherein an ith target sequence is selected, i having an initial value equal to 1; the target specific sequence in which the probes bind to the nucleic acid target sequence is then selected starting from an nth base of the selected target sequence, n having an initial value equal to 1;
(5) adding the probe binding sequence to a 5' end of the target specific sequence, and adding a reverse complementary sequence of the probe binding sequence to a 3' end of the target specific sequence; and
(6) outputting all probe sequences.

Preferably, if the specificity of the target specific sequence in which the probes bind to the nucleic acid target sequence is evaluated as high specificity, the target specific sequence is placed in the pool of probes, and spaced by adding a number m1 to the base n; and if the specificity of the target specific sequence in which the probes bind to the nucleic acid target sequence is evaluated as low specificity, the target specific sequence is not placed in the pool of probes, and spaced by adding a number m2 to the base n;

Wherein the number m1 is greater than or equal to the length of each probe and the length of the target specific sequence; and the number m2 is less than or equal to a minimum value of a length range of each probe and a length range of the target specific sequence.

Preferably, selecting the target specific sequence in which the probes bind to the nucleic acid target sequence includes the steps of: selecting a next target specific sequence when n is less than the length of the ith target sequence; and selecting the ith target specific sequence when n is greater than or equal to the length of the ith target sequence. After selection of the target specific sequence of the ith target sequence is finished, the above target specific sequence selection is performed on an i+1th target sequence until the target specific sequence selection is completed for all target sequences.

Compared with the prior art, the method of the present invention has the beneficial effects that:
(1) the processes of the hybrid capture system are optimally designed, taking into account the application requirements of an automated workstation, the connection between steps is high, the disadvantage that the conventional hybrid capture process is cumbersome is overcome, the manual operation friendliness is high, and the implementability of being applied to the automated workstation is high.
(2) The hybrid capture system has high capture efficiency, and flank sequences of the probes are complementary paired, which can significantly improve the binding ability of the probes to a target, improve the hybrid capture efficiency of the target region, and improve the overall coverage uniformity and stability.

### Brief Description of the Drawings

The accompanying drawings forming part of this application are intended to provide a further understanding of the present invention. The illustrative embodiments of the present invention and explanations thereof are intended to explain the present invention and do not constitute an improper limitation of the present invention. In the accompanying drawings:
FIG. 1 is a structural schematic diagram of probes according to the present invention, wherein each probe is mainly composed of 4 parts: a P-Cap region complementary to target genes, a P-L region at a 3' end, and a P-R region at a 5' end, wherein the 5' end of each probe is labeled with biotin, and the P-L and the P-R have a sequence complementary to each other.
FIG. 2 is a graph comparing a flow of a conventional hybrid capture system and a flow of a hybrid capture system of the present invention.
FIG. 3 is an experimental protocol for different types of samples.
FIG. 4 is a structural schematic diagram of a conventional probe of 120 nt, a short probe used in the prior art, and the probe of the present invention, wherein T represents a target fragment of a sample nucleic acid, and P represents the probe.
FIG. 5 is an experimental result of hybrid capture library NGS for the conventional probes of 120 nt, the short probes used in the prior art, and the probes of the present invention.
FIG. 6 shows the capture effect of the conventional probes of 120nt and the probes of the present invention for a PCR-free library.
FIG. 7 shows a concentration test result of the probes according to the present invention.
FIG. 8 shows a hybridization temperature test result of the probes according to the present invention.
FIG. 9 shows a hybridization time test result of the probes according to the present invention.

### Detailed Description of the Embodiments

The present invention will be further described below with reference to the accompanying drawings and specific embodiments.

All technical and scientific terms used herein have the same meaning as that commonly understood by those skilled in the art to which the present invention belongs. In other cases, certain terms used herein will have their meanings set forth in the specification. Experimental methods in which specific conditions are not indicated in the following embodiments are within the general knowledge and common general knowledge of those skilled in the art. Reagents used in the embodiments, unless otherwise specified, were purchased from reagent companies provided that the experimental requirements were met. The embodiments in this application and the features in the embodiments can be combined with each other.

The features and advantages of the present invention will be further understood from the following detailed description in conjunction with the accompanying drawings. The embodiments provided are merely illustrative of the method of the present invention, and are not intended to limit the rest of the contents of the present invention in any way.

The present invention provides a set of probes for nucleic acid capture, wherein the probes are designed separately for a positive sense strand and a negative sense strand of a target region, the probes for the positive sense strand and the probes for the negative sense strand are arranged in a non-overlapping arrangement, and a 3' or 5' end of each probe is modified with biotin which can bind to streptavidin magnetic beads.

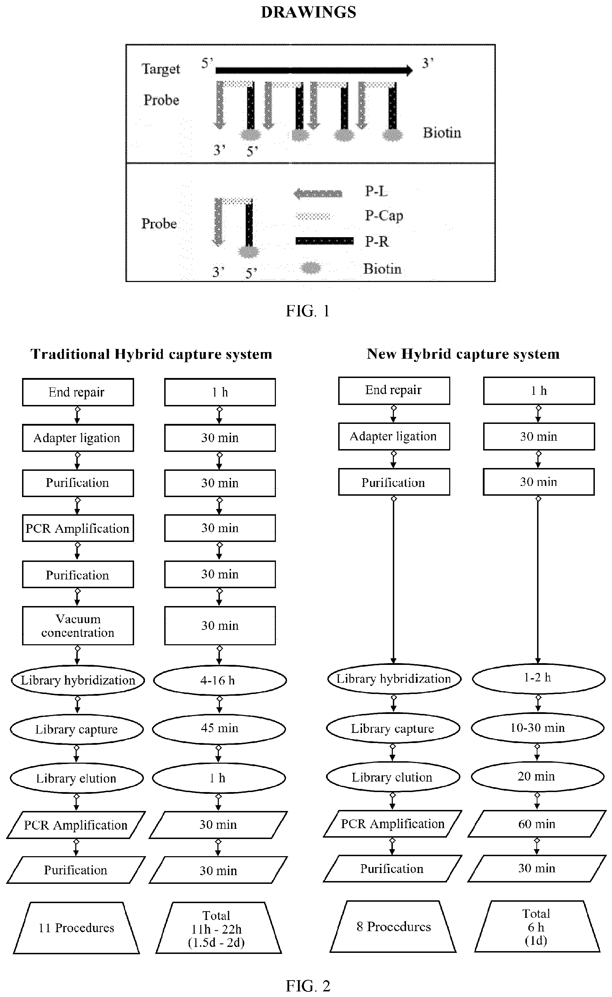

Each probe is primarily composed of three parts, wherein a middle segment is a target sequence binding segment, 5' and 3' segments are stability enhancing segments, the 5' end segment of one probe can be complementarily paired with the 3' end segment of another probe, and the 3' end segment of one probe can be complementarily paired with the 5' end segment of another probe. Fragments where the probes are complementarily paired are P-L and P-R fragments, respectively, with biotin modified at a 3' end of L or at a 5' end of R, and the biotin can bind to streptavidin on magnetic beads, and a fragment where the probes are complementarily paired with the target region is a P-Cap fragment with a P-Cap length of 20-80 nt (FIG. 1).

The probe design method is as follows:
probes are designed based on the positions of genes to be detected, namely if the probes are designed for mutation, insertion or deletion mutation, a region covering the corresponding fragment is selected to design the probes; and if the probes are designed for fusion genes, genes on both sides of a fusion gene breakpoint are selected to design the probes;
if capture of a sense strand is desired, a capture probe will be designed for the sense strand;
if capture of an antisense strand is desired, a capture probe will be designed for the antisense strand; and
by software analysis, hazardous probes are knocked out, and the hazardous probes will lead to severe off-target of the entire hybrid capture system, resulting in a reduced on-target rate, low target region capture efficiency, and poor coverage uniformity.

The present disclosure also provides a system for construction of a target library from a nucleic acid sample (see FIG. 2), wherein a specific process is as follows:
the nucleic acid sample includes a DNA sample including plasma free DNA (cfDNA), genomic DNA (gDNA), an FFPE sample, a viral or bacterial genome sample, and the like; and a RNA sample including a fresh tissue sample, an FFPE sample, a viral or bacterial genome sample, and the like.

For the cfDNA sample, without fragmenting, library construction can be performed directly;
for a complete genome sample, physical fragmenting is needed to be performed to fragment genomic DNA to about 200-250 bp;
for the RNA sample, reverse transcription, first strand synthesis and second strand synthesis are needed to be performed; and
the fragmented sample is subjected to end repair and adapter ligation, and the ligation product is purified, and the purified product is directly subjected to hybrid capture, wherein a hybrid capture solution is related to an adapter used, multi-library mixed hybridization can be performed by using a full-UDI adapter module, and the hybridization product uses Primer Mix to perform PCR amplification on the mixed hybridization captured library; if a truncated molecular tag adaptor module is used, only a single sample can be hybridized, the molecular tag-containing adaptor module can perform low-frequency mutation detection on the sample, and hybridization ambiguity and background noise introduced by the PCR amplification are filtered out through consistent sequence analysis. Here an adapter module compatible with both Illumina and MGI sequencing platforms is used to construct a DNA library suitable for different sequencing platforms.

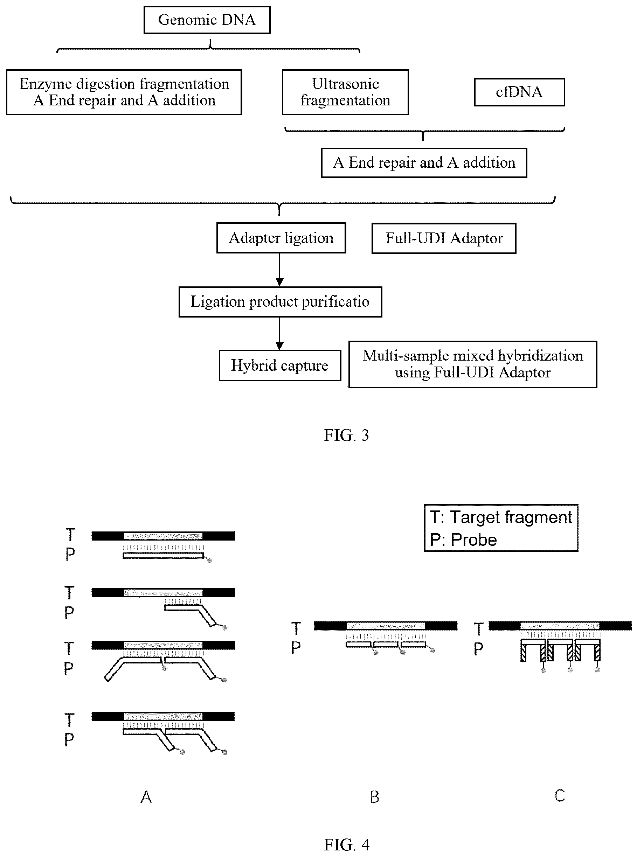

The adapter ligation product is directly used for configuring a hybrid capture reaction system without vacuum concentration, or hybrid capture can be performed directly with the adapter ligation product together with the purification magnetic beads from the previous step; and
a hybridization system uses specific probes designed in this project, so rapid hybridization can be performed. The hybridization time is 1-2 hours, and the capture time is 20 minutes, which shortens the hybrid capture time. The hybrid capture library is enriched by the PCR amplification. A PCR amplification solution in this step is related to the adaptor module used. The PCR amplification is performed in combination with primers containing a Barcode sequence when the molecular tag adaptor module is used, and the targetedly enriched DNA library is amplified in combination with Primer Mix if the full-length adaptor module is used (see FIG. 3).

The hybrid capture time selected for this system is 1h to 16h, with the most preferred capture time being 1h.

The hybrid capture temperature selected for this system is 59-61°C, and the optimal capture temperature is about 60°C, and temperature selection is related to a probe length, the GC content of a target region, and the hybrid capture time.

The construction of the hybrid capture library in this system takes a total of 6 hours from a sample to capture library obtaining, which greatly shortens the operation time of the whole process while simplifying the operation steps compared with the traditional 2-4 days.

The present disclosure also provides hybrid capture reagent components and a use method thereof, wherein the specific content is as follows:

the adapter ligation product is purified by using 2×Beads, and the purified product is treated by using a magnetic bead wash buffer configured in a kit, wherein the magnetic bead wash buffer is 4 mL of acetonitrile added into 1 mL of H₂O.

The reagents used in the hybrid capture reaction system are detailed in Table 1.

**Table 1**

| **Reagent** | **Brand** |
|---|---|
| 2×Hyb Buffer | |
| 0.01-1% BSA | Sigma |
| 0.01-1% Ficoll | Sigma |
| 0.01-1% PVP-2 | Sigma |
| 0.01-0.5 M sodium citrate | Sigma |
| 0.1-10 M NaCl | Invitrogen |
| Enhancer: 5×formamide solution | Thermo |
| Human Cot-1 1µg/µL | Thermo |
| Blocker 100 nmol | Nanodigmbio |
| pH 6.0-8.0 | |
| Probe concentration: 2-10 fmol | Nanodigmbio |

The hybridization system involves a total of 3 elution buffers, which are an elution buffer I, an elution buffer II and an elution buffer III, respectively, and formulas of the three elution buffers are shown in Table 2.

**Table 2**

| | |
|---|---|
| Elution buffer I | 5×SSPE (Sigma), 1% of SDS (Sigma) |
| Elution buffer II | 2×SSPE, 0.1% of SDS |
| Elution buffer III | 0.1×SSPE, 0.01% of SDS |

A structural schematic diagram of the probes of the present invention, a conventional probe of 120 nt and a short probe is shown in FIG. 4. The effect of the probes of the present invention is compared with the effect of a probe commonly used in the prior art in the following Examples 1-3, and the probes of the present invention are referred to NC probes.

The amount of each component configured in the kit of the present invention can be determined by those skilled in the art according to a predetermined purpose.

The kit of the present invention may also include an instruction for use. The "instruction for use" typically includes a definite recitation describing a technique employed when the components of the kit are used to achieve a desired result. Optionally, the kit may also contain other applicable components, such as a diluent, a buffer, a pharmaceutically acceptable carrier, or other applicable accessories that will be readily recognized by those skilled in the art.

**Example 1:** Comparison of a hybrid capture effect of a conventional probe of 120 bases with that of a short probe (not being a part of the present invention)

In this Example, the pre-capture library is a human plasma free DNA library derived from fragmentation and release of human genomic DNA into a blood circulation system, i.e., a sum sequence is the entire human genomic sequence. The target sequences given are located in the regions shown in Table 3, containing a series of high-frequency somatic mutation sites associated with tumors.

**Table 3 Locations of target sequences on hg19 version of human genome**

| **Chromosome** | **Target region starting point coordinate** | **Target region endpoint coordinate** | **Gene name** |
|---|---|---|---|
| chr1 | 115252204 | 115252205 | NRAS |
| chr1 | 115256518 | 115256533 | NRAS |
| chr1 | 115258730 | 115258752 | NRAS |
| chr2 | 209113106 | 209113193 | IDH1 |
| chr12 | 25378561 | 25378563 | KRAS |
| chr12 | 25378647 | 25378648 | KRAS |
| chr12 | 25380275 | 25380286 | KRAS |
| chr12 | 25398255 | 25398296 | KRAS |
| chr12 | 112888139 | 112888212 | PTPN11 |
| chr12 | 112926852 | 112926909 | PTPN11 |
| chr13 | 28592620 | 28592654 | FLT3 |
| chr13 | 28602329 | 28602330 | FLT3 |
| chr13 | 28608244 | 28608342 | FLT3 |
| chr13 | 28610138 | 28610139 | FLT3 |
| chr15 | 90631837 | 90631939 | IDH2 |
| chr17 | 7573931 | 7574027 | TP53 |
| chr17 | 7577022 | 7577146 | TP53 |
| chr17 | 7577515 | 7577606 | TP53 |
| chr17 | 7578187 | 7578293 | TP53 |
| chr17 | 7578362 | 7578559 | TP53 |
| chr17 | 7579358 | 7579474 | TP53 |
| chr17 | 7579882 | 7579883 | TP53 |

The total length of the target sequence is only 1.2 kb, and if coverage is conducted with a conventional probe of 120 nt, 44 probes are required, wherein the 44 conventional probes of 120 nt are shown in Table 4. Hybrid capture is performed with NadPrep^{®} hybrid capture reagents in this experiment, and the resulting capture library is sequenced on an Illumina Novaseq6000. In the sequencing data, 99.9% of the sequences can be mapped to a human reference genome on average, wherein 11.7% of the sequences is located in the target region on average, and the on-target rate of the probes of 120 nt is too low to meet the requirements.

**Table 4 Conventional probe of 120 nt covering the target region in Table 3 (not being a part of the present invention)**

| | **Sequence name** | **Sequence 5'-3'** | **Modificat ion** |
|---|---|---|---|
| SEQ ID NO.1 | NRAS-1 | /biotin/TGCTGAAAGCTGTACCATACCTGTCTGGTCTTGGCT GAGGTTTCAATGAATGGAATCCCGTAACTCTTGGCCAGTT CGTGGGCTTGTTTTGTATCAACTGTCCTTGTTGGCAAATCA CAC | 5' biotin |
| SEQ ID NO.2 | NRAS-2 | /biotin/TTTCAATGAATGGAATCCCGTAACTCTTGGCCAGTT CGTGGGCTTGTTTTGTATCAACTGTCCTTGTTGGCAAATCA CACTTGTTTCCCACTAGCACCATAGGTACATCATCCGAGTC TT | 5' biotin |
| SEQ ID NO.3 | NRAS-3 | /biotin/GCTATTATTGATGGCAAATACACAGAGGAAGCCTTC GCCTGTCCTCATGTATTGGTCTCTCATGGCACTGTACTCTT CTTGTCCAGCTGTATCCAGTATGTCCAACAAACAGGTTTCA CC | 5' biotin |
| SEQ ID NO.4 | NRAS-4 | /biotin/ATTGGTCTCTCATGGCACTGTACTCTTCTTGTCCAGC TGTATCCAGTATGTCCAACAAACAGGTTTCACCATCTATA ACCACTTGTTTTCTGTAAGAATCCTGGGGGTGTGGAGGGT AAG | 5' biotin |
| SEQ ID NO.5 | NRAS-5 | /biotin/TACCACTGGGCCTCACCTCTATGGTGGGATCATATT CATCTACAAAGTGGTTCTGGATTAGCTGGATTGTCAGTGC GCTTTTCCCAACACCACCTGCTCCAACCACCACCAGTTTGT ACT | 5' biotin |
| SEQ ID NO.6 | NRAS-6 | /biotin/CTCACCTCTATGGTGGGATCATATTCATCTACAAAG TGGTTCTGGATTAGCTGGATTGTCAGTGCGCTTTTCCCAAC ACCACCTGCTCCAACCACCACCAGTTTGTACTCAGTCATTT CA | 5' biotin |
| SEQ ID NO.7 | KRAS-1 | /biotin/TTTATTTCAGTGTTACTTACCTGTCTTGTCTTTGCTGA TGTTTCAATAAAAGGAATTCCATAACTTCTTGCTAAGTCCT GAGCCTGTTTTGTGTCTACTGTTCTAGAAGGCAAATCACAT | 5' biotin |
| SEQ ID NO.8 | KRAS-2 | /biotin/TTTCAATAAAAGGAATTCCATAACTTCTTGCTAAGT CCTGAGCCTGTTTTGTGTCTACTGTTCTAGAAGGCAAATCA CATTTATTTCCTACTAGGACCATAGGTACATCTTCAGAGTC CT | 5' biotin |
| SEQ ID NO.9 | KRAS-3 | /biotin/AGCCTGTTTTGTGTCTACTGTTCTAGAAGGCAAATC ACATTTATTTCCTACTAGGACCATAGGTACATCTTCAGAGT CCTTAACTCTTTTAATTTGTTCTCTGGGAAAGAAAAAAAAG TT | 5' biotin |
| SEQ ID NO.10 | KRAS-4 | /biotin/AGTATTATTTATGGCAAATACACAAAGAAAGCCCTC CCCAGTCCTCATGTACTGGTCCCTCATTGCACTGTACTCCT CTTGACCTGCTGTGTCGAGAATATCCAAGAGACAGGTTTC TCC | 5' biotin |
| SEQ ID NO.11 | KRAS-5 | /biotin/ACTGGTCCCTCATTGCACTGTACTCCTCTTGACCTGC TGTGTCGAGAATATCCAAGAGACAGGTTTCTCCATCAATT ACTACTTGCTTCCTGTAGGAATCCTGAGAAGGGAGAAACA CAG | 5' biotin |
| SEQ ID NO.12 | KRAS-6 | /biotin/TTTACCTCTATTGTTGGATCATATTCGTCCACAAAAT GATTCTGAATTAGCTGTATCGTCAAGGCACTCTTGCCTACG CCACCAGCTCCAACTACCACAAGTTTATATTCAGTCATTTT C | 5' biotin |
| SEQ ID NO.13 | KRAS-7 | /biotin/GTCCACAAAATGATTCTGAATTAGCTGTATCGTCAA GGCACTCTTGCCTACGCCACCAGCTCCAACTACCACAAGT TTATATTCAGTCATTTTCAGCAGGCCTTATAATAAAAATAA TGA | 5' biotin |
| SEQ ID NO.14 | PTPN11-1 | /biotin/TTTCCAATGGACTATTTTAGAAGAAATGGAGCTGTC ACCCACATCAAGATTCAGAACACTGGTGATTACTATGACC TGTATGGAGGGGAGAAATTTGCCACTTTGGCTGAGTTGGT CCAG | 5' biotin |
| SEQ ID NO.15 | PTPN11-2 | /biotin/ACTGGTGATTACTATGACCTGTATGGAGGGGAGAAA TTTGCCACTTTGGCTGAGTTGGTCCAGTATTACATGGAACA TCACGGGCAATTAAAAGAGAAGAATGGAGATGTCATTGA GCTT | 5' biotin |
| SEQ ID NO.16 | PTPN11-3 | /biotin/TCATGATGTTTCCTTCGTAGGTGTTGACTGCGATATT GACGTTCCCAAAACCATCCAGATGGTGCGGTCTCAGAGGT CAGGGATGGTCCAGACAGAAGCACAGTACCGATTTATCTA TAT | 5' biotin |
| SEQ ID NO.17 | PTPN11-4 | /biotin/GAGGTCAGGGATGGTCCAGACAGAAGCACAGTACC GATTTATCTATATGGCGGTCCAGCATTATATTGAAACACTA CAGCGCAGGATTGAAGAAGAGCAGGTACCAGCCTGAGGG CTGGC | 5' biotin |
| SEQ ID NO.18 | FLT3-1 | /biotin/TAGGAAATAGCAGCCTCACATTGCCCCTGACAACAT AGTTGGAATCACTCATGATATCTCGAGCCAATCCAAAGTC ACATATCTTCACCACTTTCCCGTGGGTGACAAGCACGTTCC TGG | 5' biotin |
| SEQ ID NO.19 | FLT3-2 | /biotin/TTGCCCCTGACAACATAGTTGGAATCACTCATGATA TCTCGAGCCAATCCAAAGTCACATATCTTCACCACTTTCCC GTGGGTGACAAGCACGTTCCTGGCGGCCAGGTCTCTGTGA ACA | 5' biotin |
| SEQ ID NO.20 | FLT3-3 | /biotin/GTTACCTGACAGTGTGCACGCCCCCAGCAGGTTCAC AATATTCTCGTGGCTTCCCAGCTGGGTCATCATCTTGAGTT CTGACATGAGTGCCTCTCTTTCAGAGCTGTCTGCTTTTTCT GT | 5' biotin |
| SEQ ID NO.21 | FLT3-4 | /biotin/CCCCCAGCAGGTTCACAATATTCTCGTGGCTTCCCA GCTGGGTCATCATCTTGAGTTCTGACATGAGTGCCTCTCTT TCAGAGCTGTCTGCTTTTTCTGTCAAAGAAAGGAGCATTA AAA | 5' biotin |
| SEQ ID NO.22 | FLT3-5 | /biotin/CATTCCATTCTTACCAAACTCTAAATTTTCTCTTGGA AACTCCCATTTGAGATCATATTCATATTCTCTGAAATCAAC GTAGAAGTACTCATTATCTGAGGAGCCGGTCACCTGTACC AT | 5' biotin |
| SEQ ID NO.23 | FLT3-6 | /biotin/CTAAATTTTCTCTTGGAAACTCCCATTTGAGATCATA TTCATATTCTCTGAAATCAACGTAGAAGTACTCATTATCTG AGGAGCCGGTCACCTGTACCATCTGTAGCTGGCTTTCATA CC | 5' biotin |
| SEQ ID NO.24 | FLT3-7 | /biotin/TATTACTTGGGAGACTTGTCTGAACACTTCTTCCAG GTCCAAGATGGTAATGGGTATCCATCCGAGAAACAGGACG CCTGACTTGCCGATGCTTCTGCGAGCACTTGAGGTTTCCCT ATA | 5' biotin |
| SEQ ID NO.25 | FLT3-8 | /biotin/TGAACACTTCTTCCAGGTCCAAGATGGTAATGGGTA TCCATCCGAGAAACAGGACGCCTGACTTGCCGATGCTTCT GCGAGCACTTGAGGTTTCCCTATAGAAAAGAACGTGTGAA ATAA | 5' biotin |
| SEQ ID NO.26 | IDH2-1 | /biotin/CCCTCTCCACCCTGGCCTACCTGGTCGCCATGGGCG TGCCTGCCAATGGTGATGGGCTTGGTCCAGCCAGGGACTA GGCGTGGGATGTTTTTGCAGATGATGGGCTCCCGGAAGAC AGTC | 5' biotin |
| SEQ ID NO.27 | IDH2-2 | /biotin/TGCCAATGGTGATGGGCTTGGTCCAGCCAGGGACTA GGCGTGGGATGTTTTTGCAGATGATGGGCTCCCGGAAGAC AGTCCCCCCCAGGATGTTCCGGATAGTTCCATTGGGACTTT TCC | 5' biotin |
| SEQ ID NO.28 | TP53-1 | /biotin/CACTCACCTGGAGTGAGCCCTGCTCCCCCCTGGCTC CTTCCCAGCCTGGGCATCCTTGAGTTCCAAGGCCTCATTCA GCTCTCGGAACATCTCGAAGCGCTCACGCCCACGGATCTG CAG | 5' biotin |
| SEQ ID NO.29 | TP53-2 | /biotin/TGCTCCCCCCTGGCTCCTTCCCAGCCTGGGCATCCTT GAGTTCCAAGGCCTCATTCAGCTCTCGGAACATCTCGAAG CGCTCACGCCCACGGATCTGCAGCAACAGAGGAGGGGGA GAAG | 5' biotin |
| SEQ ID NO.30 | TP53-3 | /biotin/GTGCTCCCTGGGGGCAGCTCGTGGTGAGGCTCCCCT TTCTTGCGGAGATTCTCTTCCTCTGTGCGCCGGTCTCTCCC AGGACAGGCACAAACACGCACCTCAAAGCTGTTCCGTCCC AGT | 5' biotin |
| SEQ ID NO.31 | TP53-4 | /biotin/GTGGTGAGGCTCCCCTTTCTTGCGGAGATTCTCTTCC TCTGTGCGCCGGTCTCTCCCAGGACAGGCACAAACACGCA CCTCAAAGCTGTTCCGTCCCAGTAGATTACCACTACTCAG GAT | 5' biotin |
| SEQ ID NO.32 | TP53-5 | /biotin/CTGACCTGGAGTCTTCCAGTGTGATGATGGTGAGGA TGGGCCTCCGGTTCATGCCGCCCATGCAGGAACTGTTACA CATGTAGTTGTAGTGGATGGTGGTACAGTCAGAGCCAACC TAGG | 5' biotin |
| SEQ ID NO.33 | TP53-6 | /biotin/GTGATGATGGTGAGGATGGGCCTCCGGTTCATGCCG CCCATGCAGGAACTGTTACACATGTAGTTGTAGTGGATGG TGGTACAGTCAGAGCCAACCTAGGAGATAACACAGGCCC AAGAT | 5' biotin |
| SEQ ID NO.34 | TP53-7 | /biotin/AGACCTCAGGCGGCTCATAGGGCACCACCACACTAT GTCGAAAAGTGTTTCTGTCATCCAAATACTCCACACGCAA ATTTCCTTCCACTCGGATAAGATGCTGAGGAGGGGCCAGA CCTA | 5' biotin |
| SEQ ID NO.35 | TP53-8 | /biotin/GGCACCACCACACTATGTCGAAAAGTGTTTCTGTCA TCCAAATACTCCACACGCAAATTTCCTTCCACTCGGATAA GATGCTGAGGAGGGGCCAGACCTAAGAGCAATCAGTGAG GAATC | 5' biotin |
| SEQ ID NO.36 | TP53-9 | /biotin/CTCCAGCCCCAGCTGCTCACCATCGCTATCTGAGCA GCGCTCATGGTGGGGGCAGCGCCTCACAACCTCCGTCATG TGCTGTGACTGCTTGTAGATGGCCATGGCGCGGACGCGGG TGCC | 5' biotin |
| SEQ ID NO.37 | TP53-10 | /biotin/GCCTCACAACCTCCGTCATGTGCTGTGACTGCTTGT AGATGGCCATGGCGCGGACGCGGGTGCCGGGCGGGGGTG TGGAATCAACCCACAGCTGCACAGGGCAGGTCTTGGCCAG TTGGC | 5' biotin |
| SEQ ID NO.38 | TP53-11 | /biotin/CGCGGACGCGGGTGCCGGGCGGGGGTGTGGAATCA ACCCACAGCTGCACAGGGCAGGTCTTGGCCAGTTGGCAAA ACATCTTGTTGAGGGCAGGGGAGTACTGTAGGAAGAGGA AGGAGA | 5' biotin |
| SEQ ID NO.39 | TP53-12 | /biotin/AATGCAAGAAGCCCAGACGGAAACCGTAGCTGCCC TGGTAGGTTTTCTGGGAAGGGACAGAAGATGACAGGGGC CAGGAGGGGGCTGGTGCAGGGGCCGCCGGTGTAGGAGCT GCTGGTG | 5' biotin |
| SEQ ID NO.40 | TP53-13 | /biotin/GAAGGGACAGAAGATGACAGGGGCCAGGAGGGGG CTGGTGCAGGGGCCGCCGGTGTAGGAGCTGCTGGTGCAGG GGCCACGGGGGGAGCAGCCTCTGGCATTCTGGGAGCTTCA TCTGGA | 5' biotin |
| SEQ ID NO.41 | TP53-14 | /biotin/GCCCTTCCAATGGATCCACTCACAGTTTCCATAGGT CTGAAAATGTTTCCTGACTCAGAGGGGGCTCGACGCTAGG ATCTGACTGCGGCTCCTCCATGGCAGTGACCCGGAAGGCA GTCT | 5' biotin |
| SEQ ID NO.42 | TP53-15 | /biotin/CACAGTTTCCATAGGTCTGAAAATGTTTCCTGACTC AGAGGGGGCTCGACGCTAGGATCTGACTGCGGCTCCTCCA TGGCAGTGACCCGGAAGGCAGTCTGGCTGCTGCAAGAGG AAAAG | 5' biotin |
| SEQ ID NO.43 | IDH1-1 | /biotin/TTATTGCCAACATGACTTACTTGATCCCCATAAGCA TGACGACCTATGATGATAGGTTTTACCCATCCACTCACAA GCCGGGGGATATTTTTGCAGATAATGGCTTCTCTGAAGAC CGTG | 5' biotin |
| SEQ ID NO.44 | IDH1-2 | /biotin/AGGTTTTACCCATCCACTCACAAGCCGGGGGATATT TTTGCAGATAATGGCTTCTCTGAAGACCGTGCCACCCAGA ATATTTCGTATGGTGCCATTTGGTGATTTCCACATTTGTTTC AA | 5' biotin |

The most concentrated length distribution of plasma free DNA fragments is about 160bp, so there may be not a probe capable of binding to a plasma free DNA fragment completely, and the overall binding of the probes to the target sequence is not stable. Furthermore, the proportion of the target region to the whole genome is very small, only about 1/2500000, and a low on-target rate result can also be expected.

To increase the probability of binding each fragment to probes, short probes are employed for capture. With a shorter probe length, it is hoped that there are 4 probes to which each fragment to be enriched of 160 bp can bind, i.e. the probe length does not exceed 40 nt. The target annealing temperature of each probe is set at 65°C. The annealing temperature is greatly influenced by a sequence base composition if the probe length is shorter, so the design method for the pool of probes is different from that of the conventional probes of 120 nt, and it is necessary to adjust the probe length within a certain range to make its annealing temperature close to a target value. Design of the pool of probes is performed according to part of the steps of the design method for the pool of probes provided by the present disclosure (a, d, g, preferably m1=40, and m2=5), the sum sequence is the human reference genome hg19, the target sequence is a target region sequence as shown in Table 3, and a probe length range parameter of 35-40 nt, and the probe annealing temperature of 65°C are input. The resulting short probes are shown in Table 5, approximately 40 bp in length, with a total of 97 probes. After capture library NGS data analysis, it is shown that 99.9% of the sequences can be mapped to the human reference genome on average, wherein 23.4% of the sequences is located in the target region on average. Although there is a significant increase in the on-target rate, the on-target rate is still less than the requirement of conventional hybrid capture on the on-target rate of 50%. It is obvious that even if the probes are shortened directly, and the probe density is increased in overlapping probes for capture, the on-target rate cannot reach the basic requirement of 50%.

**Table 5 Short probes covering the target region in Table 3 (not being a part of the present invention)**

| | **Sequence name** | **Sequence 5'-3'** | **Modifica tion** |
|---|---|---|---|
| SEQ ID NO.45 | NRAS-S-1 | /biotin/CAAATGCTGAAAGCTGTACCATACCTGTCTGGT CT | 5' biotin |
| SEQ ID NO.46 | NRAS-S-2 | /biotin/GCTGAGGTTTCAATGAATGGAATCCCGTAACTC TT | 5' biotin |
| SEQ ID NO.47 | NRAS-S-3 | /biotin/CCAGTTCGTGGGCTTGTTTGTATCAACTGTCCT T | 5' biotin |
| SEQ ID NO.48 | NRAS-S-4 | /biotin/TGGCAAATCACACTTGTTTCCCACTAGCACCATA G | 5' biotin |
| SEQ ID NO.49 | NRAS-S-5 | /biotin/ACATCATCCGAGTCTTTTACTCGCTTAATCTGCT C | 5' biotin |
| SEQ ID NO.50 | NRAS-S-6 | /biotin/ACTTGCTATTATTGATGGCAAATACACAGAGGA AGCC | 5' biotin |
| SEQ ID NO.51 | NRAS-S-7 | /biotin/CGCCTGTCCTCATGTATTGGTCTCTCATGGCACT G | 5' biotin |
| SEQ ID NO.52 | NRAS-S-8 | /biotin/CTCTTCTTGTCCAGCTGTATCCAGTATGTCCAAC A | 5' biotin |
| SEQ ID NO.53 | NRAS-S-9 | /biotin/CAGGTTTCACCATCTATAACCACTTGTTTTCTGT AAGAAT | 5' biotin |
| SEQ ID NO.54 | NRAS-S-10 | /biotin/CCTGGGGGTGTGGAGGGTAAGGGGGCAGGGAG GGA | 5' biotin |
| SEQ ID NO.55 | NRAS-S-11 | /biotin/GGGCTACCACTGGGCCTCACCTCTATGGTGGGA TC | 5' biotin |
| SEQ ID NO.56 | NRAS-S-12 | /biotin/ATTCATCTACAAAGTGGTTCTGGATTAGCTGGAT TGTC | 5' biotin |
| SEQ ID NO.57 | NRAS-S-13 | /biotin/TGCGCTTTTCCCAACACCACCTGCTCCAACCACC A | 5' biotin |
| SEQ ID NO.58 | NRAS-S-14 | /biotin/AGTTTGTACTCAGTCATTTCACACCAGCAAGAA CC | 5' biotin |
| SEQ ID NO.59 | KRAS-S-1 | /biotin/TATTTATTTCAGTGTTACTTACCTGTCTTGTCTTT GCTGA | 5' biotin |
| SEQ ID NO.60 | KRAS-S-2 | /biotin/TGTTTCAATAAAAGGAATTCCATAACTTCTTGCT AAGTCC | 5' biotin |
| SEQ ID NO.61 | KRAS-S-3 | /biotin/TGAGCCTGTTTGTGTCTACTGTTCTAGAAGGCA A | 5' biotin |
| SEQ ID NO.62 | KRAS-S-4 | /biotin/CACATTTATTTCCTACTAGGACCATAGGTACATC TTCAG | 5' biotin |
| SEQ ID NO.63 | KRAS-S-5 | /biotin/GTCCTTAACTCTTTTAATTTGTTCTCTGGGAAAG AAAAAA | 5' biotin |
| SEQ ID NO.64 | KRAS-S-6 | /biotin/AAGTTATAGCACAGTCATTAGTAACACAAATAT CTTTCAA | 5' biotin |
| SEQ ID NO.65 | KRAS-S-7 | /biotin/TAGTATTATTTATGGCAAATACACAAAGAAAGC CCTCCCC | 5' biotin |
| SEQ ID NO.66 | KRAS-S-8 | /biotin/AGTCCTCATGTACTGGTCCCTCATTGCACTGTAC T | 5' biotin |
| SEQ ID NO.67 | KRAS-S-9 | /biotin/TCTTGACCTGCTGTGTCGAGAATATCCAAGAGA CA | 5' biotin |
| SEQ ID NO.68 | KRAS-S-10 | /biotin/TTTCTCCATCAATTACTACTTGCTTCCTGTAGGA ATCC | 5' biotin |
| SEQ ID NO.69 | KRAS-S-11 | /biotin/AGAAGGGAGAAACACAGTCTGGATTATTACAGT GC | 5' biotin |
| SEQ ID NO.70 | KRAS-S-12 | /biotin/GATTTACCTCTATTGTTGGATCATATTCGTCCAC AAAATG | 5' biotin |
| SEQ ID NO.71 | KRAS-S-13 | /biotin/ATTCTGAATTAGCTGTATCGTCAAGGCACTCTTG C | 5' biotin |
| SEQ ID NO.72 | KRAS-S-14 | /biotin/ACGCCACCAGCTCCAACTACCACAAGTTTATAT TC | 5' biotin |
| SEQ ID NO.73 | KRAS-S-15 | /biotin/TCATTTTCAGCAGGCCTTATAATAAAAATAATG AAAATGT | 5' biotin |
| SEQ ID NO.74 | PTPN11-S-1 | /biotin/CTTTCCAATGGACTATTTTAGAAGAAATGGAGC TGTCAC | 5' biotin |
| SEQ ID NO.75 | PTPN11-S-2 | /biotin/CACATCAAGATTCAGAACACTGGTGATTACTAT GACC | 5' biotin |
| SEQ ID NO.76 | PTPN11-S-3 | /biotin/TATGGAGGGGAGAAATTTGCCACTTTGGCTGAG TT | 5' biotin |
| SEQ ID NO.77 | PTPN11-S-4 | /biotin/TCCAGTATTACATGGAACATCACGGGCAATTAA AAGAG | 5' biotin |
| SEQ ID NO.78 | PTPN11-S-5 | /biotin/GAATGGAGATGTCATTGAGCTTAAATATCCTCT GAACTG | 5' biotin |
| SEQ ID NO.79 | PTPN11-S-6 | /biotin/CTTCATGATGTTTCCTTCGTAGGTGTTGACTGCG A | 5' biotin |
| SEQ ID NO.80 | PTPN11-S-7 | /biotin/TTGACGTTCCCAAAACCATCCAGATGGTGCGGT CT | 5' biotin |
| SEQ ID NO.81 | PTPN11-S-8 | /biotin/GTACCGATTTATCTATATGGCGGTCCAGCATTAT ATTG | 5' biotin |
| SEQ ID NO.82 | PTPN11-S-9 | /biotin/ACACTACAGCGCAGGATTGAAGAAGAGCAGGT ACC | 5' biotin |
| SEQ ID NO.83 | PTPN11-S-1 0 | /biotin/CCTGAGGGCTGGCATGCGGATTCTCATTCTCTTG C | 5' biotin |
| SEQ ID NO.84 | FLT3-S-1 | /biotin/TAAGTAGGAAATAGCAGCCTCACATTGCCCCTG AC | 5' biotin |
| SEQ ID NO.85 | FLT3-S-2 | /biotin/CATAGTTGGAATCACTCATGATATCTCGAGCCA ATC | 5' biotin |
| SEQ ID NO.86 | FLT3-S-3 | /biotin/AAGTCACATATCTTCACCACTTTCCCGTGGGTGA C | 5' biotin |
| SEQ ID NO.87 | FLT3-S-4 | /biotin/GCACGTTCCTGGCGGCCAGGTCTCTGTGAACAC AC | 5' biotin |
| SEQ ID NO.88 | FLT3-S-5 | /biotin/GTGGGTTACCTGACAGTGTGCACGCCCCCAGCA GG | 5' biotin |
| SEQ ID NO.89 | FLT3-S-6 | /biotin/CACAATATTCTCGTGGCTTCCCAGCTGGGTCATC A | 5' biotin |
| SEQ ID NO.90 | FLT3-S-7 | /biotin/TTGAGTTCTGACATGAGTGCCTCTCTTTCAGAGC T | 5' biotin |
| SEQ ID NO.91 | FLT3-S-8 | /biotin/CTGCTTTTCTGTCAAAGAAAGGAGCATTAAAA ATGTAAA | 5' biotin |
| SEQ ID NO.92 | FLT3-S-9 | /biotin/GGCACATTCCATTCTTACCAAACTCTAAATTTTC TCTTGG | 5' biotin |
| SEQ ID NO.93 | FLT3-S-10 | /biotin/AAACTCCCATTTGAGATCATATTCATATTCTCTG AAATCA | 5' biotin |
| SEQ ID NO.94 | FLT3-S-11 | /biotin/ACGTAGAAGTACTCATTATCTGAGGAGCCGGTC AC | 5' biotin |
| SEQ ID NO.95 | FLT3-S-12 | /biotin/GTACCATCTGTAGCTGGCTTTCATACCTAAATTG C | 5' biotin |
| SEQ ID NO.96 | FLT3-S-13 | /biotin/TATTACTTGGGAGACTTGTCTGAACACTTCTTCC AG | 5' biotin |
| SEQ ID NO.97 | FLT3-S-14 | /biotin/CCAAGATGGTAATGGGTATCCATCCGAGAAACA GG | 5' biotin |
| SEQ ID NO.98 | FLT3-S-15 | /biotin/GCCTGACTTGCCGATGCTTCTGCGAGCACTTGA GG | 5' biotin |
| SEQ ID NO.99 | FLT3-S-16 | /biotin/TCCCTATAGAAAAGAACGTGTGAAATAAGCTCA CTGG | 5' biotin |
| SEQ ID NO.100 | IDH2-S-1 | /biotin/ATCCCCTCTCCACCCTGGCCTACCTGGTCGCCAT G | 5' biotin |
| SEQ ID NO.101 | IDH2-S-2 | /biotin/CGTGCCTGCCAATGGTGATGGGCTTGGTCCAGC CA | 5' biotin |
| SEQ ID NO.102 | IDH2-S-3 | /biotin/GACTAGGCGTGGATGTTTTTGCAGATGATGGG CT | 5' biotin |
| SEQ ID NO.103 | IDH2-S-4 | /biotin/CGGAAGACAGTCCCCCCCAGGATGTTCCGGATA GT | 5' biotin |
| SEQ ID NO.104 | IDH2-S-5 | /biotin/CATTGGGACTTTTCCACATCTTCTTCAGCTTGAA C | 5' biotin |
| SEQ ID NO.105 | TP53-S-1 | /biotin/AGGTCACTCACCTGGAGTGAGCCCTGCTCCCCC CT | 5' biotin |
| SEQ ID NO.106 | TP53-S-2 | /biotin/CTCCTTCCCAGCCTGGGCATCCTTGAGTTCCAAG G | 5' biotin |
| SEQ ID NO.107 | TP53-S-3 | /biotin/TCATTCAGCTCTCGGAACATCTCGAAGCGCTCA CG | 5' biotin |
| SEQ ID NO.108 | TP53-S-4 | /biotin/CACGGATCTGCAGCAACAGAGGAGGGGGAGAA GTA | 5' biotin |
| SEQ ID NO.109 | TP53-S-5 | /biotin/AGTGCTCCCTGGGGGCAGCTCGTGGTGAGGCTC CC | 5' biotin |
| SEQ ID NO.110 | TP53-S-6 | /biotin/TTCTTGCGGAGATTCTCTTCCTCTGTGCGCCGGT C | 5' biotin |
| SEQ ID NO.111 | TP53-S-7 | /biotin/TCCCAGGACAGGCACAAACACGCACCTCAAAGC TG | 5' biotin |
| SEQ ID NO.112 | TP53-S-8 | /biotin/CCGTCCCAGTAGATTACCACTACTCAGGATAGG AA | 5' biotin |
| SEQ ID NO.113 | TP53-S-9 | /biotin/CTCCTGACCTGGAGTCTTCCAGTGTGATGATGGT G | 5' biotin |
| SEQ ID NO.114 | TP53-S-10 | /biotin/GATGGGCCTCCGGTTCATGCCGCCCATGCAGGA AC | 5' biotin |
| SEQ ID NO.115 | TP53-S-11 | /biotin/TTACACATGTAGTTGTAGTGGATGGTGGTACAG TC | 5' biotin |
| SEQ ID NO.116 | TP53-S-12 | /biotin/AGCCAACCTAGGAGATAACACAGGCCCAAGAT GAG | 5' biotin |
| SEQ ID NO.117 | TP53-S-13 | /biotin/CCAGACCTCAGGCGGCTCATAGGGCACCACCAC AC | 5' biotin |
| SEQ ID NO.118 | TP53-S-14 | /biotin/TGTCGAAAAGTGTTTCTGTCATCCAAATACTCCA CAC | 5' biotin |
| SEQ ID NO.119 | TP53-S-15 | /biotin/AAATTTCCTTCCACTCGGATAAGATGCTGAGGA GG | 5' biotin |
| SEQ ID NO.120 | TP53-S-16 | /biotin/CCAGACCTAAGAGCAATCAGTGAGGAATCAGA GGC | 5' biotin |
| SEQ ID NO.121 | TP53-S-17 | /biotin/CTCCAGCCCCAGCTGCTCACCATCGCTATCTGAG C | 5' biotin |
| SEQ ID NO.122 | TP53-S-18 | /biotin/CGCTCATGGTGGGGGCAGCGCCTCACAACCTCC GT | 5' biotin |
| SEQ ID NO.123 | TP53-S-19 | /biotin/TGTGCTGTGACTGCTTGTAGATGGCCATGGCGC GG | 5' biotin |
| SEQ ID NO.124 | TP53-S-20 | /biotin/GCGGGTGCCGGCGGGGTGTGGAATCAACCCA CA | 5' biotin |
| SEQ ID NO.125 | TP53-S-21 | /biotin/TGCACAGGGCAGGTCTTGGCCAGTTGGCAAAAC AT | 5' biotin |
| SEQ ID NO.126 | TP53-S-22 | /biotin/TGTTGAGGGCAGGGGAGTACTGTAGGAAGAGG AAG | 5' biotin |
| SEQ ID NO.127 | TP53-S-23 | /biotin/GACAGAGTTGAAAGTCAGGGCACAAGTGAACA GAT | 5' biotin |
| SEQ ID NO.128 | TP53-S-24 | /biotin/AATGCAAGAAGCCCAGACGGAAACCGTAGCTG CCC | 5' biotin |
| SEQ ID NO.129 | TP53-S-25 | /biotin/GTAGGTTTCTGGGAAGGGACAGAAGATGACAG GG | 5' biotin |
| SEQ ID NO.130 | TP53-S-26 | /biotin/CAGGAGGGGCTGGTGCAGGGCCGCCGGTGT AGG | 5' biotin |
| SEQ ID NO.131 | TP53-S-27 | /biotin/CTGCTGGTGCAGGGGCCACGGGGGAGCAGCCT CT | 5' biotin |
| SEQ ID NO.132 | TP53-S-28 | /biotin/CATTCTGGGAGCTTCATCTGGACCTGGGTCTTCA G | 5' biotin |
| SEQ ID NO.133 | TP53-S-29 | /biotin/GCCCTTCCAATGGATCCACTCACAGTTTCCATAG G | 5' biotin |
| SEQ ID NO.134 | TP53-S-30 | /biotin/TGAAAATGTTTCCTGACTCAGAGGGGGCTCGAC GC | 5' biotin |
| SEQ ID NO.135 | TP53-S-31 | /biotin/GGATCTGACTGCGGCTCCTCCATGGCAGTGACC CG | 5' biotin |
| SEQ ID NO.136 | TP53-S-32 | /biotin/AGGCAGTCTGGCTGCTGCAAGAGGAAAAGTGG GGA | 5' biotin |
| SEQ ID NO.137 | IDH1-S-1 | /biotin/CATTATTGCCAACATGACTTACTTGATCCCCATA AGC | 5' biotin |
| SEQ ID NO.138 | IDH1-S-2 | /biotin/GACGACCTATGATGATAGGTTTTACCCATCCACT C | 5' biotin |
| SEQ ID NO.139 | IDH1-S-3 | /biotin/AAGCCGGGGGATATTTTTGCAGATAATGGCTTC TC | 5' biotin |
| SEQ ID NO.140 | IDH1-S-4 | /biotin/AAGACCGTGCCACCCAGAATATTTCGTATGGTG CC | 5' biotin |
| SEQ ID NO.141 | IDH1-S-5 | /biotin/TTGGTGATTTCCACATTTGTTTCAACTTGAACTC CTCAAC | 5' biotin |

**Example 2:** Comparison of a hybrid capture effect of conventional probes of 120 bp (not being a part of the present invention) with that of the NC probes (according to the present invention)

In this Example, comparison of the capture results of a human plasma free DNA library by the NC probes and conventional probes of 120bp with the capture results of the same target region in Example 1 is shown.

The NC probes are probes in which sequences for probes binding to each other are added to the short probe sequences shown in Table 5. According to the design method for the pool of probes provided by the present invention, the sum sequence is the human reference genome hg19, the target sequences are the target region sequence as shown in Table 3, the probe length range is set as 35-40 nt, and the probe annealing temperature is set as 65°C. The sequence length of a region wherein probes bind to each other is set as 8, i.e., k=8. A total of 65536 of all possible sequence combinations of 8 bases occur in the human reference genome hg19, with an average number of occurrences of 88419. From sequences with the lower number of occurrences, the selected probe binding sequence is CGTCGGTC, and its complementary sequence is GACCGACG, with a number of occurrences of 2078. This sequence is added to both sides of the probes in Table 5 as the probe binding sequence.

**Table 6 NC probes covering the target region in Table 3**

| | **Sequence name** | **Sequence 5'-3'** | **Modific ation** |
|---|---|---|---|
| SEQ ID NO.142 | NRAS-NC-1 | /biotin/CGTCGGTCCAAATGCTGAAAGCTGTACCATACCTG TCTGGTCTGACCGACG | 5' biotin |
| SEQ ID NO.143 | NRAS-NC-2 | /biotin/CGTCGGTCGCTGAGGTTTCAATGAATGGAATCCCG TAACTCTTGACCGACG | 5' biotin |
| SEQ ID NO.144 | NRAS-NC-3 | /biotin/CGTCGGTCCCAGTTCGTGGGCTTGTTTTGTATCAA CTGTCCTTGACCGACG | 5' biotin |
| SEQ ID NO.145 | NRAS-NC-4 | /biotin/CGTCGGTCTGGCAAATCACACTTGTTTCCCACTAG CACCATAGGACCGACG | 5' biotin |
| SEQ ID NO.146 | NRAS-NC-5 | /biotin/CGTCGGTCACATCATCCGAGTCTTTTACTCGCTTA ATCTGCTCGACCGACG | 5' biotin |
| SEQ ID NO.147 | NRAS-NC-6 | /biotin/CGTCGGTCACTTGCTATTATTGATGGCAAATACAC AGAGGAAGCCGACCGACG | 5' biotin |
| SEQ ID NO.148 | NRAS-NC-7 | /biotin/CGTCGGTCCGCCTGTCCTCATGTATTGGTCTCTCAT GGCACTGGACCGACG | 5' biotin |
| SEQ ID NO.149 | NRAS-NC-8 | /biotin/CGTCGGTCCTCTTCTTGTCCAGCTGTATCCAGTAT GTCCAACAGACCGACG | 5' biotin |
| SEQ ID NO.150 | NRAS-NC-9 | /biotin/CGTCGGTCCAGGTTTCACCATCTATAACCACTTGT TTTCTGTAAGAATGACCGACG | 5' biotin |
| SEQ ID NO.151 | NRAS-NC-10 | /biotin/CGTCGGTCCCTGGGGGTGTGGAGGGTAAGGGGGC AGGGAGGGAGACCGACG | 5' biotin |
| SEQ ID NO.152 | NRAS-NC-11 | /biotin/CGTCGGTCGGGCTACCACTGGGCCTCACCTCTATG GTGGGATCGACCGACG | 5' biotin |
| SEQ ID NO.153 | NRAS-NC-12 | /biotin/CGTCGGTCATTCATCTACAAAGTGGTTCTGGATTA GCTGGATTGTCGACCGACG | 5' biotin |
| SEQ ID NO.154 | NRAS-NC-13 | /biotin/CGTCGGTCTGCGCTTTTCCCAACACCACCTGCTCC AACCACCAGACCGACG | 5' biotin |
| SEQ ID NO.155 | NRAS-NC-14 | /biotin/CGTCGGTCAGTTTGTACTCAGTCATTTCACACCAG CAAGAACCGACCGACG | 5' biotin |
| SEQ ID NO.156 | KRAS-NC-1 | /biotin/CGTCGGTCTATTTATTTCAGTGTTACTTACCTGTCT TGTCTTTGCTGAGACCGACG | 5' biotin |
| SEQ ID NO.157 | KRAS-NC-2 | /biotin/CGTCGGTCTGTTTCAATAAAAGGAATTCCATAACT TCTTGCTAAGTCCGACCGACG | 5' biotin |
| SEQ ID NO.158 | KRAS-NC-3 | /biotin/CGTCGGTCTGAGCCTGTTTTGTGTCTACTGTTCTAG AAGGCAAGACCGACG | 5' biotin |
| SEQ ID NO.159 | KRAS-NC-4 | /biotin/CGTCGGTCCACATTTATTTCCTACTAGGACCATAG GTACATCTTCAGGACCGACG | 5' biotin |
| SEQ ID NO.160 | KRAS-NC-5 | /biotin/CGTCGGTCGTCCTTAACTCTTTTAATTTGTTCTCTG GGAAAGAAAAAAGACCGACG | 5' biotin |
| SEQ ID NO.161 | KRAS-NC-6 | /biotin/CGTCGGTCAAGTTATAGCACAGTCATTAGTAACAC AAATATCTTTCAAGACCGACG | 5' biotin |
| SEQ ID NO.162 | KRAS-NC-7 | /biotin/CGTCGGTCTAGTATTATTTATGGCAAATACACAAA GAAAGCCCTCCCCGACCGACG | 5' biotin |
| SEQ ID NO.163 | KRAS-NC-8 | /biotin/CGTCGGTCAGTCCTCATGTACTGGTCCCTCATTGC ACTGTACTGACCGACG | 5' biotin |
| SEQ ID NO.164 | KRAS-NC-9 | /biotin/CGTCGGTCTCTTGACCTGCTGTGTCGAGAATATCC AAGAGACAGACCGACG | 5' biotin |
| SEQ ID NO.165 | KRAS-NC-10 | /biotin/CGTCGGTCTTTCTCCATCAATTACTACTTGCTTCCT GTAGGAATCCGACCGACG | 5' biotin |
| SEQ ID NO.166 | KRAS-NC-11 | /biotin/CGTCGGTCAGAAGGGAGAAACACAGTCTGGATTA TTACAGTGCGACCGACG | 5' biotin |
| SEQ ID NO.167 | KRAS-NC-12 | /biotin/CGTCGGTCGATTTACCTCTATTGTTGGATCATATTC GTCCACAAAATGGACCGACG | 5' biotin |
| SEQ ID NO.168 | KRAS-NC-13 | /biotin/CGTCGGTCATTCTGAATTAGCTGTATCGTCAAGGC ACTCTTGCGACCGACG | 5' biotin |
| SEQ ID NO.169 | KRAS-NC-14 | /biotin/CGTCGGTCACGCCACCAGCTCCAACTACCACAAG TTTATATTCGACCGACG | 5' biotin |
| SEQ ID NO.170 | KRAS-NC-15 | /biotin/CGTCGGTCTCATTTTCAGCAGGCCTTATAATAAAA ATAATGAAAATGTGACCGACG | 5' biotin |
| SEQ ID NO.171 | PTPN11-NC-1 | /biotin/CGTCGGTCCTTTCCAATGGACTATTTTAGAAGAAA TGGAGCTGTCACGACCGACG | 5' biotin |
| SEQ ID NO.172 | PTPN11-NC-2 | /biotin/CGTCGGTCCACATCAAGATTCAGAACACTGGTGAT TACTATGACCGACCGACG | 5' biotin |
| SEQ ID NO.173 | PTPN11-NC-3 | /biotin/CGTCGGTCTATGGAGGGGAGAAATTTGCCACTTTG GCTGAGTTGACCGACG | 5' biotin |
| SEQ ID NO.174 | PTPN11-NC-4 | /biotin/CGTCGGTCTCCAGTATTACATGGAACATCACGGGC AATTAAAAGAGGACCGACG | 5' biotin |
| SEQ ID NO.175 | PTPN11-NC-5 | /biotin/CGTCGGTCGAATGGAGATGTCATTGAGCTTAAATA TCCTCTGAACTGGACCGACG | 5' biotin |
| SEQ ID NO.176 | PTPN11-NC-6 | /biotin/CGTCGGTCCTTCATGATGTTTCCTTCGTAGGTGTTG ACTGCGAGACCGACG | 5' biotin |
| SEQ ID NO. 177 | PTPN11-NC-7 | /biotin/CGTCGGTCTTGACGTTCCCAAAACCATCCAGATGG TGCGGTCTGACCGACG | 5' biotin |
| SEQ ID NO.178 | PTPN11-NC-8 | /biotin/CGTCGGTCGTACCGATTTATCTATATGGCGGTCCA GCATTATATTGGACCGACG | 5' biotin |
| SEQ ID NO.179 | PTPN11-NC-9 | /biotin/CGTCGGTCACACTACAGCGCAGGATTGAAGAAGA GCAGGTACCGACCGACG | 5' biotin |
| SEQ ID NO.180 | PTPN11-NC-1 0 | /biotin/CGTCGGTCCCTGAGGGCTGGCATGCGGATTCTCAT TCTCTTGCGACCGACG | 5' biotin |
| SEQ ID NO.181 | FLT3-NC-1 | /biotin/CGTCGGTCTAAGTAGGAAATAGCAGCCTCACATT GCCCCTGACGACCGACG | 5' biotin |
| SEQ ID NO.182 | FLT3-NC-2 | /biotin/CGTCGGTCCATAGTTGGAATCACTCATGATATCTC GAGCCAATCGACCGACG | 5' biotin |
| SEQ ID NO.183 | FLT3-NC-3 | /biotin/CGTCGGTCAAGTCACATATCTTCACCACTTTCCCG TGGGTGACGACCGACG | 5' biotin |
| SEQ ID NO.184 | FLT3-NC-4 | /biotin/CGTCGGTCGCACGTTCCTGGCGGCCAGGTCTCTGT GAACACACGACCGACG | 5' biotin |
| SEQ ID NO.185 | FLT3-NC-5 | /biotin/CGTCGGTCGTGGGTTACCTGACAGTGTGCACGCCC CCAGCAGGGACCGACG | 5' biotin |
| SEQ ID NO.186 | FLT3-NC-6 | /biotin/CGTCGGTCCACAATATTCTCGTGGCTTCCCAGCTG GGTCATCAGACCGACG | 5' biotin |
| SEQ ID NO.187 | FLT3-NC-7 | /biotin/CGTCGGTCTTGAGTTCTGACATGAGTGCCTCTCTT TCAGAGCTGACCGACG | 5' biotin |
| SEQ ID NO.188 | FLT3-NC-8 | /biotin/CGTCGGTCCTGCTTTTTCTGTCAAAGAAAGGAGCA TTAAAAATGTAAAGACCGACG | 5' biotin |
| SEQ ID NO.189 | FLT3-NC-9 | /biotin/CGTCGGTCGGCACATTCCATTCTTACCAAACTCTA AATTTTCTCTTGGGACCGACG | 5' biotin |
| SEQ ID NO.190 | FLT3-NC-10 | /biotin/CGTCGGTCAAACTCCCATTTGAGATCATATTCATA TTCTCTGAAATCAGACCGACG | 5' biotin |
| SEQ ID NO.191 | FLT3-NC-11 | /biotin/CGTCGGTCACGTAGAAGTACTCATTATCTGAGGAG CCGGTCACGACCGACG | 5' biotin |
| SEQ ID NO.192 | FLT3-NC-12 | /biotin/CGTCGGTCGTACCATCTGTAGCTGGCTTTCATACC TAAATTGCGACCGACG | 5' biotin |
| SEQ ID NO.193 | FLT3-NC-13 | /biotin/CGTCGGTCTATTACTTGGGAGACTTGTCTGAACAC TTCTTCCAGGACCGACG | 5' biotin |
| SEQ ID NO.194 | FLT3-NC-14 | /biotin/CGTCGGTCCCAAGATGGTAATGGGTATCCATCCGA GAAACAGGGACCGACG | 5' biotin |
| SEQ ID NO.195 | FLT3-NC-15 | /biotin/CGTCGGTCGCCTGACTTGCCGATGCTTCTGCGAGC ACTTGAGGGACCGACG | 5' biotin |
| SEQ ID NO.196 | FLT3-NC-16 | /biotin/CGTCGGTCTCCCTATAGAAAAGAACGTGTGAAAT AAGCTCACTGGGACCGACG | 5' biotin |
| SEQ ID NO.197 | IDH2-NC-1 | /biotin/CGTCGGTCATCCCCTCTCCACCCTGGCCTACCTGG TCGCCATGGACCGACG | 5' biotin |
| SEQ ID NO.198 | IDH2-NC-2 | /biotin/CGTCGGTCCGTGCCTGCCAATGGTGATGGGCTTGG TCCAGCCAGACCGACG | 5' biotin |
| SEQ ID NO.199 | IDH2-NC-3 | /biotin/CGTCGGTCGACTAGGCGTGGGATGTTTTTGCAGAT GATGGGCTGACCGACG | 5' biotin |
| SEQ ID NO.200 | IDH2-NC-4 | /biotin/CGTCGGTCCGGAAGACAGTCCCCCCCAGGATGTTC CGGATAGTGACCGACG | 5' biotin |
| SEQ ID NO.201 | IDH2-NC-5 | /biotin/CGTCGGTCCATTGGGACTTTTCCACATCTTCTTCA GCTTGAACGACCGACG | 5' biotin |
| SEQ ID NO.202 | TP53-NC-1 | /biotin/CGTCGGTCAGGTCACTCACCTGGAGTGAGCCCTGC TCCCCCCTGACCGACG | 5' biotin |
| SEQ ID NO.203 | TP53-NC-2 | /biotin/CGTCGGTCCTCCTTCCCAGCCTGGGCATCCTTGAG TTCCAAGGGACCGACG | 5' biotin |
| SEQ ID NO.204 | TP53-NC-3 | /biotin/CGTCGGTCTCATTCAGCTCTCGGAACATCTCGAAG CGCTCACGGACCGACG | 5' biotin |
| SEQ ID NO.205 | TP53-NC-4 | /biotin/CGTCGGTCCACGGATCTGCAGCAACAGAGGAGGG GGAGAAGTAGACCGACG | 5' biotin |
| SEQ ID NO.206 | TP53-NC-5 | /biotin/CGTCGGTCAGTGCTCCCTGGGGGCAGCTCGTGGTG AGGCTCCCGACCGACG | 5' biotin |
| SEQ ID NO.207 | TP53-NC-6 | /biotin/CGTCGGTCTTCTTGCGGAGATTCTCTTCCTCTGTGC GCCGGTCGACCGACG | 5' biotin |
| SEQ ID NO.208 | TP53-NC-7 | /biotin/CGTCGGTCTCCCAGGACAGGCACAAACACGCACC TCAAAGCTGGACCGACG | 5' biotin |
| SEQ ID NO.209 | TP53-NC-8 | /biotin/CGTCGGTCCCGTCCCAGTAGATTACCACTACTCAG GATAGGAAGACCGACG | 5' biotin |
| SEQ ID NO.210 | TP53-NC-9 | /biotin/CGTCGGTCCTCCTGACCTGGAGTCTTCCAGTGTGA TGATGGTGGACCGACG | 5' biotin |
| SEQ ID NO.211 | TP53-NC-10 | /biotin/CGTCGGTCGATGGGCCTCCGGTTCATGCCGCCCAT GCAGGAACGACCGACG | 5' biotin |
| SEQ ID NO.212 | TP53-NC-11 | /biotin/CGTCGGTCTTACACATGTAGTTGTAGTGGATGGTG GTACAGTCGACCGACG | 5' biotin |
| SEQ ID NO.213 | TP53-NC-12 | /biotin/CGTCGGTCAGCCAACCTAGGAGATAACACAGGCC CAAGATGAGGACCGACG | 5' biotin |
| SEQ ID NO.214 | TP53-NC-13 | /biotin/CGTCGGTCCCAGACCTCAGGCGGCTCATAGGGCA CCACCACACGACCGACG | 5' biotin |
| SEQ ID NO.215 | TP53-NC-14 | /biotin/CGTCGGTCTGTCGAAAAGTGTTTCTGTCATCCAAA TACTCCACACGACCGACG | 5' biotin |
| SEQ ID NO.216 | TP53-NC-15 | /biotin/CGTCGGTCAAATTTCCTTCCACTCGGATAAGATGC TGAGGAGGGACCGACG | 5' biotin |
| SEQ ID NO.217 | TP53-NC-16 | /biotin/CGTCGGTCCCAGACCTAAGAGCAATCAGTGAGGA ATCAGAGGCGACCGACG | 5' biotin |
| SEQ ID NO.218 | TP53-NC-17 | /biotin/CGTCGGTCCTCCAGCCCCAGCTGCTCACCATCGCT ATCTGAGCGACCGACG | 5' biotin |
| SEQ ID NO.219 | TP53-NC-18 | /biotin/CGTCGGTCCGCTCATGGTGGGGGCAGCGCCTCAC AACCTCCGTGACCGACG | 5' biotin |
| SEQ ID NO.220 | TP53-NC-19 | /biotin/CGTCGGTCTGTGCTGTGACTGCTTGTAGATGGCCA TGGCGCGGGACCGACG | 5' biotin |
| SEQ ID NO.221 | TP53-NC-20 | /biotin/CGTCGGTCGCGGGTGCCGGGCGGGGGTGTGGAAT CAACCCACAGACCGACG | 5' biotin |
| SEQ ID NO.222 | TP53-NC-21 | /biotin/CGTCGGTCTGCACAGGGCAGGTCTTGGCCAGTTGG CAAAACATGACCGACG | 5' biotin |
| SEQ ID NO.223 | TP53-NC-22 | /biotin/CGTCGGTCTGTTGAGGGCAGGGGAGTACTGTAGG AAGAGGAAGGACCGACG | 5' biotin |
| SEQ ID NO.224 | TP53-NC-23 | /biotin/CGTCGGTCGACAGAGTTGAAAGTCAGGGCACAAG TGAACAGATGACCGACG | 5' biotin |
| SEQ ID NO.225 | TP53-NC-24 | /biotin/CGTCGGTCAATGCAAGAAGCCCAGACGGAAACCG TAGCTGCCCGACCGACG | 5' biotin |
| SEQ ID NO.226 | TP53-NC-25 | /biotin/CGTCGGTCGTAGGTTTTCTGGGAAGGGACAGAAG ATGACAGGGGACCGACG | 5' biotin |
| SEQ ID NO.227 | TP53-NC-26 | /biotin/CGTCGGTCCAGGAGGGGGCTGGTGCAGGGGCCGC CGGTGTAGGGACCGACG | 5' biotin |
| SEQ ID NO.228 | TP53-NC-27 | /biotin/CGTCGGTCCTGCTGGTGCAGGGGCCACGGGGGGA GCAGCCTCTGACCGACG | 5' biotin |
| SEQ ID NO.229 | TP53-NC-28 | /biotin/CGTCGGTCCATTCTGGGAGCTTCATCTGGACCTGG GTCTTCAGGACCGACG | 5' biotin |
| SEQ ID NO.230 | TP53-NC-29 | /biotin/CGTCGGTCGCCCTTCCAATGGATCCACTCACAGTT TCCATAGGGACCGACG | 5' biotin |
| SEQ ID NO.231 | TP53-NC-30 | /biotin/CGTCGGTCTGAAAATGTTTCCTGACTCAGAGGGGG CTCGACGCGACCGACG | 5' biotin |
| SEQ ID NO.232 | TP53-NC-31 | /biotin/CGTCGGTCGGATCTGACTGCGGCTCCTCCATGGCA GTGACCCGGACCGACG | 5' biotin |
| SEQ ID NO.233 | TP53-NC-32 | /biotin/CGTCGGTCAGGCAGTCTGGCTGCTGCAAGAGGAA AAGTGGGGAGACCGACG | 5' biotin |
| SEQ ID NO.234 | IDH1-NC-1 | /biotin/CGTCGGTCCATTATTGCCAACATGACTTACTTGAT CCCCATAAGCGACCGACG | 5' biotin |
| SEQ ID NO.235 | IDH1-NC-2 | /biotin/CGTCGGTCGACGACCTATGATGATAGGTTTTACCC ATCCACTCGACCGACG | 5' biotin |
| SEQ ID NO.236 | IDH1-NC-3 | /biotin/CGTCGGTCAAGCCGGGGGATATTTTTGCAGATAAT GGCTTCTCGACCGACG | 5' biotin |
| SEQ ID NO.237 | IDH1-NC-4 | /biotin/CGTCGGTCAAGACCGTGCCACCCAGAATATTTCGT ATGGTGCCGACCGACG | 5' biotin |
| SEQ ID NO.238 | IDH1-NC-5 | /biotin/CGTCGGTCTTGGTGATTTCCACATTTGTTTCAACTT GAACTCCTCAACGACCGACG | 5' biotin |

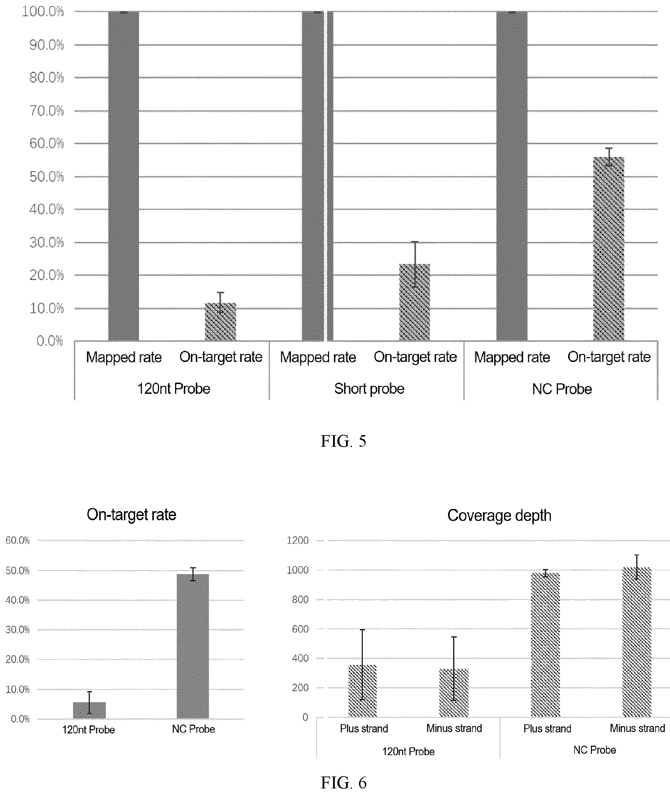

The results are shown in FIG. 5, NGS data of the NC probe captured library shows that 99.9% of the sequences can be mapped to the human reference genome, and an average proportion of sequences located in the target regions is 56.0%, which meets the on-target rate requirements in conventional hybrid capture.

### Example 3: Targeted capture of a PCR-free library using NC probes

A PCR-free library refers to a library that is connected to a NGS adapter, but is not subjected to PCR amplification, wherein original sequence information is retained, and PCR preferences are not introduced. Hybrid capture with the PCR-free library directly suffers from the difficulties of low hybridization input and an unguaranteed capture rate. After PCR amplification of the library, each original fragment has multiple copies, so there are multiple opportunities to be bound and captured by probes. If any fragment in the PCR-free library is not captured by the probes, it cannot enter the next step, resulting in information loss. Moreover, after PCR, each single strand of the library fragment generates a corresponding complementary strand, so the probes only need to be designed in one direction to capture information from both strands of the original fragment. Whereas in the PCR-free library, both positive and negative strands of one fragment are present singly, and if the probes in only one direction are used for capture, the complementary chains will also be lost. Thus, in this Example, a probe of the other strand is added. The other strand probes for the conventional probes of 120 bp are shown in Table 7, and the other strand probes for the NC probes are shown in Table 8.

As shown in FIG. 6, after 30 ng of a plasma free DNA PCR-free library is captured by the conventional probes of 120 bp in Tables 4 and 7, the NGS results show an average on-target rate of only 5.6%, an average coverage depth of 356.1x of plus strands after deduplication, and an average depth of 329.9x of minus strands after deduplication. Whereas after capture by the NC probes shown in Tables 6 and 8, the NGS results show that the average on-target rate reaches 48.7%, with an average depth of 980.2x of the plus strands after deduplication, and an average depth of 1020.5x of the minus strands after deduplication. It can be seen that for the PCR-free library, the recovery rate and the on-target rate for the NC probes are greatly improved.

**Table 7 Complementary strand probes for the conventional probes of 120bp covering the target region in Table 3 (not being a part of the present invention)**

| | **Sequence name** | **Sequence 5'-3'** | **Modifica tion** |
|---|---|---|---|
| SEQ ID NO.239 | NRAS-OP-1 | /biotin/GTGTGATTTGCCAACAAGGACAGTTGATACAAAAC AAGCCCACGAACTGGCCAAGAGTTACGGGATTCCATTCA TTGAAACCTCAGCCAAGACCAGACAGGTATGGTACAGCT TTCAGCA | 5' biotin |
| SEQ ID NO.240 | NRAS-OP-2 | /biotin/AAGACTCGGATGATGTACCTATGGTGCTAGTGGGA AACAAGTGTGATTTGCCAACAAGGACAGTTGATACAAAA CAAGCCCACGAACTGGCCAAGAGTTACGGGATTCCATTC ATTGAAA | 5' biotin |
| SEQ ID NO.241 | NRAS-OP-3 | /biotin/GGTGAAACCTGTTTGTTGGACATACTGGATACAGCT GGACAAGAAGAGTACAGTGCCATGAGAGACCAATACATG AGGACAGGCGAAGGCTTCCTCTGTGTATTTGCCATCAATA ATAGC | 5' biotin |
| SEQ ID NO.242 | NRAS-OP-4 | /biotin/CTTACCCTCCACACCCCCAGGATTCTTACAGAAAAC AAGTGGTTATAGATGGTGAAACCTGTTTGTTGGACATACT GGATACAGCTGGACAAGAAGAGTACAGTGCCATGAGAGA CCAAT | 5' biotin |
| SEQ ID NO.243 | NRAS-OP-5 | /biotin/AGTACAAACTGGTGGTGGTTGGAGCAGGTGGTGTT GGGAAAAGCGCACTGACAATCCAGCTAATCCAGAACCAC TTTGTAGATGAATATGATCCCACCATAGAGGTGAGGCCCA GTGGTA | 5' biotin |
| SEQ ID NO.244 | NRAS-OP-6 | /biotin/TGAAATGACTGAGTACAAACTGGTGGTGGTTGGAG CAGGTGGTGTTGGGAAAAGCGCACTGACAATCCAGCTAA TCCAGAACCACTTTGTAGATGAATATGATCCCACCATAGA GGTGAG | 5' biotin |
| SEQ ID NO.245 | KRAS-OP-1 | /biotin/ATGTGATTTGCCTTCTAGAACAGTAGACACAAAAC AGGCTCAGGACTTAGCAAGAAGTTATGGAATTCCTTTTAT TGAAACATCAGCAAAGACAAGACAGGTAAGTAACACTGA | 5' biotin |
| | | AATAAA | |
| SEQ ID NO.246 | KRAS-OP-2 | /biotin/AGGACTCTGAAGATGTACCTATGGTCCTAGTAGGA AATAAATGTGATTTGCCTTCTAGAACAGTAGACACAAAA CAGGCTCAGGACTTAGCAAGAAGTTATGGAATTCCTTTTA TTGAAA | 5' biotin |
| SEQ ID NO.247 | KRAS-OP-3 | /biotin/AACTTTTTTTTCTTTCCCAGAGAACAAATTAAAAGAG TTAAGGACTCTGAAGATGTACCTATGGTCCTAGTAGGAAA TAAATGTGATTTGCCTTCTAGAACAGTAGACACAAAACA GGCT | 5' biotin |
| SEQ ID NO.248 | KRAS-OP-4 | /biotin/GGAGAAACCTGTCTCTTGGATATTCTCGACACAGC AGGTCAAGAGGAGTACAGTGCAATGAGGGACCAGTACAT GAGGACTGGGGAGGGCTTTCTTTGTGTATTTGCCATAAAT AATACT | 5' biotin |
| SEQ ID NO.249 | KRAS-OP-5 | /biotin/CTGTGTTTCTCCCTTCTCAGGATTCCTACAGGAAGC AAGTAGTAATTGATGGAGAAACCTGTCTCTTGGATATTCT CGACACAGCAGGTCAAGAGGAGTACAGTGCAATGAGGGA CCAGT | 5' biotin |
| SEQ ID NO.250 | KRAS-OP-6 | /biotin/GAAAATGACTGAATATAAACTTGTGGTAGTTGGAG CTGGTGGCGTAGGCAAGAGTGCCTTGACGATACAGCTAA TTCAGAATCATTTTGTGGACGAATATGATCCAACAATAGA GGTAAA | 5' biotin |
| SEQ ID NO.251 | KRAS-OP-7 | /biotin/TCATTATTTTTATTATAAGGCCTGCTGAAAATGACT GAATATAAACTTGTGGTAGTTGGAGCTGGTGGCGTAGGC AAGAGTGCCTTGACGATACAGCTAATTCAGAATCATTTTG TGGAC | 5' biotin |
| SEQ ID NO.252 | PTPN11-OP -1 | /biotin/CTGGACCAACTCAGCCAAAGTGGCAAATTTCTCCC CTCCATACAGGTCATAGTAATCACCAGTGTTCTGAATCTT GATGTGGGTGACAGCTCCATTTCTTCTAAAATAGTCCATT GGAAA | 5' biotin |
| SEQ ID NO.253 | PTPN11-OP -2 | /biotin/AAGCTCAATGACATCTCCATTCTTCTCTTTTAATTG CCCGTGATGTTCCATGTAATACTGGACCAACTCAGCCAAA GTGGCAAATTTCTCCCCTCCATACAGGTCATAGTAATCAC CAGT | 5' biotin |
| SEQ ID NO.254 | PTPN11-OP -3 | /biotin/ATATAGATAAATCGGTACTGTGCTTCTGTCTGGACC ATCCCTGACCTCTGAGACCGCACCATCTGGATGGTTTTGG GAACGTCAATATCGCAGTCAACACCTACGAAGGAAACAT CATGA | 5' biotin |
| SEQ ID NO.255 | PTPN11-OP -4 | /biotin/GCCAGCCCTCAGGCTGGTACCTGCTCTTCTTCAATC CTGCGCTGTAGTGTTTCAATATAATGCTGGACCGCCATAT AGATAAATCGGTACTGTGCTTCTGTCTGGACCATCCCTGA CCTC | 5' biotin |
| SEQ ID NO.256 | FLT3-OP-1 | /biotin/CCAGGAACGTGCTTGTCACCCACGGGAAAGTGGTG AAGATATGTGACTTTGGATTGGCTCGAGATATCATGAGTG ATTCCAACTATGTTGTCAGGGGCAATGTGAGGCTGCTATT TCCTA | 5' biotin |
| SEQ ID NO.257 | FLT3-OP-2 | /biotin/TGTTCACAGAGACCTGGCCGCCAGGAACGTGCTTG TCACCCACGGGAAAGTGGTGAAGATATGTGACTTTGGATT GGCTCGAGATATCATGAGTGATTCCAACTATGTTGTCAGG GGCAA | 5' biotin |
| SEQ ID NO.258 | FLT3-OP-3 | /biotin/ACAGAAAAAGCAGACAGCTCTGAAAGAGAGGCAC TCATGTCAGAACTCAAGATGATGACCCAGCTGGGAAGCC ACGAGAATATTGTGAACCTGCTGGGGGCGTGCACACTGT CAGGTAAC | 5' biotin |
| SEQ ID NO.259 | FLT3-OP-4 | /biotin/TTTTAATGCTCCTTTCTTTGACAGAAAAAGCAGACA GCTCTGAAAGAGAGGCACTCATGTCAGAACTCAAGATGA TGACCCAGCTGGGAAGCCACGAGAATATTGTGAACCTGC TGGGGG | 5' biotin |
| SEQ ID NO.260 | FLT3-OP-5 | /biotin/ATGGTACAGGTGACCGGCTCCTCAGATAATGAGTA CTTCTACGTTGATTTCAGAGAATATGAATATGATCTCAAA TGGGAGTTTCCAAGAGAAAATTTAGAGTTTGGTAAGAAT GGAATG | 5' biotin |
| SEQ ID NO.261 | FLT3-OP-6 | /biotin/GGTATGAAAGCCAGCTACAGATGGTACAGGTGACC GGCTCCTCAGATAATGAGTACTTCTACGTTGATTTCAGAG AATATGAATATGATCTCAAATGGGAGTTTCCAAGAGAAA ATTTAG | 5' biotin |
| SEQ ID NO.262 | FLT3-OP-7 | /biotin/TATAGGGAAACCTCAAGTGCTCGCAGAAGCATCGG CAAGTCAGGCGTCCTGTTTCTCGGATGGATACCCATTACC ATCTTGGACCTGGAAGAAGTGTTCAGACAAGTCTCCCAA GTAATA | 5' biotin |
| SEQ ID NO.263 | FLT3-OP-8 | /biotin/TTATTTCACACGTTCTTTTCTATAGGGAAACCTCAA GTGCTCGCAGAAGCATCGGCAAGTCAGGCGTCCTGTTTCT CGGATGGATACCCATTACCATCTTGGACCTGGAAGAAGT GTTCA | 5' biotin |
| SEQ ID NO.264 | IDH2-OP-1 | /biotin/GACTGTCTTCCGGGAGCCCATCATCTGCAAAAACA TCCCACGCCTAGTCCCTGGCTGGACCAAGCCCATCACCAT TGGCAGGCACGCCCATGGCGACCAGGTAGGCCAGGGTGG AGAGGG | 5' biotin |
| SEQ ID NO.265 | IDH2-OP-2 | /biotin/GGAAAAGTCCCAATGGAACTATCCGGAACATCCTG GGGGGGACTGTCTTCCGGGAGCCCATCATCTGCAAAAAC ATCCCACGCCTAGTCCCTGGCTGGACCAAGCCCATCACCA TTGGCA | 5' biotin |
| SEQ ID NO.266 | TP53-OP-1 | /biotin/CTGCAGATCCGTGGGCGTGAGCGCTTCGAGATGTT CCGAGAGCTGAATGAGGCCTTGGAACTCAAGGATGCCCA GGCTGGGAAGGAGCCAGGGGGGAGCAGGGCTCACTCCAG GTGAGTG | 5' biotin |
| SEQ ID NO.267 | TP53-OP-2 | /biotin/CTTCTCCCCCTCCTCTGTTGCTGCAGATCCGTGGGC GTGAGCGCTTCGAGATGTTCCGAGAGCTGAATGAGGCCTT GGAACTCAAGGATGCCCAGGCTGGGAAGGAGCCAGGGG GGAGCA | 5' biotin |
| SEQ ID NO.268 | TP53-OP-3 | /biotin/ACTGGGACGGAACAGCTTTGAGGTGCGTGTTTGTG CCTGTCCTGGGAGAGACCGGCGCACAGAGGAAGAGAATC TCCGCAAGAAAGGGGAGCCTCACCACGAGCTGCCCCCAG GGAGCAC | 5' biotin |
| SEQ ID NO.269 | TP53-OP-4 | /biotin/ATCCTGAGTAGTGGTAATCTACTGGGACGGAACAG CTTTGAGGTGCGTGTTTGTGCCTGTCCTGGGAGAGACCGG CGCACAGAGGAAGAGAATCTCCGCAAGAAAGGGGAGCCT CACCAC | 5' biotin |
| SEQ ID NO.270 | TP53-OP-5 | /biotin/CCTAGGTTGGCTCTGACTGTACCACCATCCACTACA ACTACATGTGTAACAGTTCCTGCATGGGCGGCATGAACCG GAGGCCCATCCTCACCATCATCACACTGGAAGACTCCAG GTCAG | 5' biotin |
| SEQ ID NO.271 | TP53-OP-6 | /biotin/ATCTTGGGCCTGTGTTATCTCCTAGGTTGGCTCTGA CTGTACCACCATCCACTACAACTACATGTGTAACAGTTCC TGCATGGGCGGCATGAACCGGAGGCCCATCCTCACCATC ATCAC | 5' biotin |
| SEQ ID NO.272 | TP53-OP-7 | /biotin/TAGGTCTGGCCCCTCCTCAGCATCTTATCCGAGTGG AAGGAAATTTGCGTGTGGAGTATTTGGATGACAGAAACA CTTTTCGACATAGTGTGGTGGTGCCCTATGAGCCGCCTGA GGTCT | 5' biotin |
| SEQ ID NO.273 | TP53-OP-8 | /biotin/GATTCCTCACTGATTGCTCTTAGGTCTGGCCCCTCC TCAGCATCTTATCCGAGTGGAAGGAAATTTGCGTGTGGAG TATTTGGATGACAGAAACACTTTTCGACATAGTGTGGTGG TGCC | 5' biotin |
| SEQ ID NO.274 | TP53-OP-9 | /biotin/GGCACCCGCGTCCGCGCCATGGCCATCTACAAGCA GTCACAGCACATGACGGAGGTTGTGAGGCGCTGCCCCCA CCATGAGCGCTGCTCAGATAGCGATGGTGAGCAGCTGGG GCTGGAG | 5' biotin |
| SEQ ID NO.275 | TP53-OP-10 | /biotin/GCCAACTGGCCAAGACCTGCCCTGTGCAGCTGTGG GTTGATTCCACACCCCCGCCCGGCACCCGCGTCCGCGCCA TGGCCATCTACAAGCAGTCACAGCACATGACGGAGGTTG TGAGGC | 5' biotin |
| SEQ ID NO.276 | TP53-OP-11 | /biotin/TCTCCTTCCTCTTCCTACAGTACTCCCCTGCCCTCAA CAAGATGTTTTGCCAACTGGCCAAGACCTGCCCTGTGCAG CTGTGGGTTGATTCCACACCCCCGCCCGGCACCCGCGTCC GCG | 5' biotin |
| SEQ ID NO.277 | TP53-OP-12 | /biotin/CACCAGCAGCTCCTACACCGGCGGCCCCTGCACCA GCCCCCTCCTGGCCCCTGTCATCTTCTGTCCCTTCCCAGAA AACCTACCAGGGCAGCTACGGTTTCCGTCTGGGCTTCTTG CATT | 5' biotin |
| SEQ ID NO.278 | TP53-OP-13 | /biotin/TCCAGATGAAGCTCCCAGAATGCCAGAGGCTGCTC CCCCCGTGGCCCCTGCACCAGCAGCTCCTACACCGGCGGC CCCTGCACCAGCCCCCTCCTGGCCCCTGTCATCTTCTGTCC CTTC | 5' biotin |
| SEQ ID NO.279 | TP53-OP-14 | /biotin/AGACTGCCTTCCGGGTCACTGCCATGGAGGAGCCG CAGTCAGATCCTAGCGTCGAGCCCCCTCTGAGTCAGGAA ACATTTTCAGACCTATGGAAACTGTGAGTGGATCCATTGG AAGGGC | 5' biotin |
| SEQ ID NO.280 | TP53-OP-15 | /biotin/CTTTTCCTCTTGCAGCAGCCAGACTGCCTTCCGGGT CACTGCCATGGAGGAGCCGCAGTCAGATCCTAGCGTCGA GCCCCCTCTGAGTCAGGAAACATTTTCAGACCTATGGAAA CTGTG | 5' biotin |
| SEQ ID NO.281 | IDH1-OP-1 | /biotin/CACGGTCTTCAGAGAAGCCATTATCTGCAAAAATA TCCCCCGGCTTGTGAGTGGATGGGTAAAACCTATCATCAT AGGTCGTCATGCTTATGGGGATCAAGTAAGTCATGTTGGC AATAA | 5' biotin |
| SEQ ID NO.282 | IDH1-OP-2 | /biotin/TTGAAACAAATGTGGAAATCACCAAATGGCACCAT ACGAAATATTCTGGGTGGCACGGTCTTCAGAGAAGCCATT ATCTGCAAAAATATCCCCCGGCTTGTGAGTGGATGGGTAA AACCT | 5' biotin |

**Table 8 Complementary strand probes for the NC probes covering the target region in Table 3**

| | **Sequence name** | **Sequence 5'-3'** | **Modific ation** |
|---|---|---|---|
| SEQ ID NO.283 | NRAS-NC-OP -1 | /biotin/CGTCGGTCAGACCAGACAGGTATGGTACAGCTTTC AGCATTTGGACCGACG | 5' biotin |
| SEQ ID NO.284 | NRAS-NC-OP -2 | /biotin/CGTCGGTCAAGAGTTACGGGATTCCATTCATTGAA ACCTCAGCGACCGACG | 5' biotin |
| SEQ ID NO.285 | NRAS-NC-OP -3 | /biotin/CGTCGGTCAAGGACAGTTGATACAAAACAAGCCC ACGAACTGGGACCGACG | 5' biotin |
| SEQ ID NO.286 | NRAS-NC-OP -4 | /biotin/CGTCGGTCCTATGGTGCTAGTGGGAAACAAGTGTG ATTTGCCAGACCGACG | 5' biotin |
| SEQ ID NO.287 | NRAS-NC-OP -5 | /biotin/CGTCGGTCGAGCAGATTAAGCGAGTAAAAGACTC GGATGATGTGACCGACG | 5' biotin |
| SEQ ID NO.288 | NRAS-NC-OP -6 | /biotin/CGTCGGTCGGCTTCCTCTGTGTATTTGCCATCAATA ATAGCAAGTGACCGACG | 5' biotin |
| SEQ ID NO.289 | NRAS-NC-OP -7 | /biotin/CGTCGGTCCAGTGCCATGAGAGACCAATACATGA GGACAGGCGGACCGACG | 5' biotin |
| SEQ ID NO.290 | NRAS-NC-OP -8 | /biotin/CGTCGGTCTGTTGGACATACTGGATACAGCTGGAC AAGAAGAGGACCGACG | 5' biotin |
| SEQ ID NO.291 | NRAS-NC-OP -9 | /biotin/CGTCGGTCATTCTTACAGAAAACAAGTGGTTATAG ATGGTGAAACCTGGACCGACG | 5' biotin |
| SEQ ID NO.292 | NRAS-NC-OP -10 | /biotin/CGTCGGTCTCCCTCCCTGCCCCCTTACCCTCCACAC CCCCAGGGACCGACG | 5' biotin |
| SEQ ID NO.293 | NRAS-NC-OP -11 | /biotin/CGTCGGTCGATCCCACCATAGAGGTGAGGCCCAGT GGTAGCCCGACCGACG | 5' biotin |
| SEQ ID NO.294 | NRAS-NC-OP -12 | /biotin/CGTCGGTCGACAATCCAGCTAATCCAGAACCACTT TGTAGATGAATGACCGACG | 5' biotin |
| SEQ ID NO.295 | NRAS-NC-OP -13 | /biotin/CGTCGGTCTGGTGGTTGGAGCAGGTGGTGTTGGGA AAAGCGCAGACCGACG | 5' biotin |
| SEQ ID NO.296 | NRAS-NC-OP -14 | /biotin/CGTCGGTCGGTTCTTGCTGGTGTGAAATGACTGAG TACAAACTGACCGACG | 5' biotin |
| SEQ ID NO.297 | KRAS-NC-OP -1 | /biotin/CGTCGGTCTCAGCAAAGACAAGACAGGTAAGTAA CACTGAAATAAATAGACCGACG | 5' biotin |
| SEQ ID NO.298 | KRAS-NC-OP -2 | /biotin/CGTCGGTCGGACTTAGCAAGAAGTTATGGAATTCC TTTTATTGAAACAGACCGACG | 5' biotin |
| SEQ ID NO.299 | KRAS-NC-OP -3 | /biotin/CGTCGGTCTTGCCTTCTAGAACAGTAGACACAAAA CAGGCTCAGACCGACG | 5' biotin |
| SEQ ID NO.300 | KRAS-NC-OP -4 | /biotin/CGTCGGTCCTGAAGATGTACCTATGGTCCTAGTAG GAAATAAATGTGGACCGACG | 5' biotin |
| SEQ ID NO.301 | KRAS-NC-OP -5 | /biotin/CGTCGGTCTTTTTTCTTTCCCAGAGAACAAATTAA AAGAGTTAAGGACGACCGACG | 5' biotin |
| SEQ ID NO.302 | KRAS-NC-OP -6 | /biotin/CGTCGGTCTTGAAAGATATTTGTGTTACTAATGAC TGTGCTATAACTTGACCGACG | 5' biotin |
| SEQ ID NO.303 | KRAS-NC-OP -7 | /biotin/CGTCGGTCGGGGAGGGCTTTCTTTGTGTATTTGCC ATAAATAATACTAGACCGACG | 5' biotin |
| SEQ ID NO.304 | KRAS-NC-OP -8 | /biotin/CGTCGGTCAGTACAGTGCAATGAGGGACCAGTAC ATGAGGACTGACCGACG | 5' biotin |
| SEQ ID NO.305 | KRAS-NC-OP -9 | /biotin/CGTCGGTCTGTCTCTTGGATATTCTCGACACAGCA GGTCAAGAGACCGACG | 5' biotin |
| SEQ ID NO.306 | KRAS-NC-OP -10 | /biotin/CGTCGGTCGGATTCCTACAGGAAGCAAGTAGTAAT TGATGGAGAAAGACCGACG | 5' biotin |
| SEQ ID NO.307 | KRAS-NC-OP -11 | /biotin/CGTCGGTCGCACTGTAATAATCCAGACTGTGTTTC TCCCTTCTGACCGACG | 5' biotin |
| SEQ ID NO.308 | KRAS-NC-OP -12 | /biotin/CGTCGGTCCATTTTGTGGACGAATATGATCCAACA ATAGAGGTAAATCGACCGACG | 5' biotin |
| SEQ ID NO.309 | KRAS-NC-OP -13 | /biotin/CGTCGGTCGCAAGAGTGCCTTGACGATACAGCTAA TTCAGAATGACCGACG | 5' biotin |
| SEQ ID NO.310 | KRAS-NC-OP -14 | /biotin/CGTCGGTCGAATATAAACTTGTGGTAGTTGGAGCT GGTGGCGTGACCGACG | 5' biotin |
| SEQ ID NO.311 | KRAS-NC-OP -15 | /biotin/CGTCGGTCACATTTTCATTATTTTTATTATAAGGCC TGCTGAAAATGAGACCGACG | 5' biotin |
| SEQ ID NO.312 | PTPN11-NC-OP-1 | /biotin/CGTCGGTCGTGACAGCTCCATTTCTTCTAAAATAG TCCATTGGAAAGGACCGACG | 5' biotin |
| SEQ ID NO.313 | PTPN11-NC-OP-2 | /biotin/CGTCGGTCGGTCATAGTAATCACCAGTGTTCTGAA TCTTGATGTGGACCGACG | 5' biotin |
| SEQ ID NO.314 | PTPN11-NC-OP-3 | /biotin/CGTCGGTCAACTCAGCCAAAGTGGCAAATTTCTCC CCTCCATAGACCGACG | 5' biotin |
| SEQ ID NO.315 | PTPN11-NC-OP-4 | /biotin/CGTCGGTCCTCTTTTAATTGCCCGTGATGTTCCATG TAATACTGGAGACCGACG | 5' biotin |
| SEQ ID NO.316 | PTPN11-NC-OP-5 | /biotin/CGTCGGTCCAGTTCAGAGGATATTTAAGCTCAATG ACATCTCCATTCGACCGACG | 5' biotin |
| SEQ ID NO.317 | PTPN11-NC-OP-6 | /biotin/CGTCGGTCTCGCAGTCAACACCTACGAAGGAAAC ATCATGAAGGACCGACG | 5' biotin |
| SEQ ID NO.318 | PTPN11-NC-OP-7 | /biotin/CGTCGGTCAGACCGCACCATCTGGATGGTTTTGGG AACGTCAAGACCGACG | 5' biotin |
| SEQ ID NO.319 | PTPN11-NC-OP-8 | /biotin/CGTCGGTCCAATATAATGCTGGACCGCCATATAGA TAAATCGGTACGACCGACG | 5' biotin |
| SEQ ID NO.320 | PTPN11-NC-OP-9 | /biotin/CGTCGGTCGGTACCTGCTCTTCTTCAATCCTGCGCT GTAGTGTGACCGACG | 5' biotin |
| SEQ ID NO.321 | PTPN11-NC-OP-10 | /biotin/CGTCGGTCGCAAGAGAATGAGAATCCGCATGCCA GCCCTCAGGGACCGACG | 5' biotin |
| SEQ ID NO.322 | FLT3-NC-OP-1 | /biotin/CGTCGGTCGTCAGGGGCAATGTGAGGCTGCTATTT CCTACTTAGACCGACG | 5' biotin |
| SEQ ID NO.323 | FLT3-NC-OP-2 | /biotin/CGTCGGTCGATTGGCTCGAGATATCATGAGTGATT CCAACTATGGACCGACG | 5' biotin |
| SEQ ID NO.324 | FLT3-NC-OP-3 | /biotin/CGTCGGTCGTCACCCACGGGAAAGTGGTGAAGAT ATGTGACTTGACCGACG | 5' biotin |
| SEQ ID NO.325 | FLT3-NC-OP-4 | /biotin/CGTCGGTCGTGTGTTCACAGAGACCTGGCCGCCAG GAACGTGCGACCGACG | 5' biotin |
| SEQ ID NO.326 | FLT3-NC-OP-5 | /biotin/CGTCGGTCCCTGCTGGGGGCGTGCACACTGTCAGG TAACCCACGACCGACG | 5' biotin |
| SEQ ID NO.327 | FLT3-NC-OP-6 | /biotin/CGTCGGTCTGATGACCCAGCTGGGAAGCCACGAG AATATTGTGGACCGACG | 5' biotin |
| SEQ ID NO.328 | FLT3-NC-OP-7 | /biotin/CGTCGGTCAGCTCTGAAAGAGAGGCACTCATGTCA GAACTCAAGACCGACG | 5' biotin |
| SEQ ID NO.329 | FLT3-NC-OP-8 | /biotin/CGTCGGTCTTTACATTTTTAATGCTCCTTTCTTTGA CAGAAAAAGCAGGACCGACG | 5' biotin |
| SEQ ID NO.330 | FLT3-NC-OP-9 | /biotin/CGTCGGTCCCAAGAGAAAATTTAGAGTTTGGTAAG AATGGAATGTGCCGACCGACG | 5' biotin |
| SEQ ID NO.331 | FLT3-NC-OP-10 | /biotin/CGTCGGTCTGATTTCAGAGAATATGAATATGATCT CAAATGGGAGTTTGACCGACG | 5' biotin |
| SEQ ID NO.332 | FLT3-NC-OP-11 | /biotin/CGTCGGTCGTGACCGGCTCCTCAGATAATGAGTAC TTCTACGTGACCGACG | 5' biotin |
| SEQ ID NO.333 | FLT3-NC-OP-12 | /biotin/CGTCGGTCGCAATTTAGGTATGAAAGCCAGCTACA GATGGTACGACCGACG | 5' biotin |
| SEQ ID NO.334 | FLT3-NC-OP-13 | /biotin/CGTCGGTCCTGGAAGAAGTGTTCAGACAAGTCTCC CAAGTAATAGACCGACG | 5' biotin |
| SEQ ID NO.335 | FLT3-NC-OP-14 | /biotin/CGTCGGTCCCTGTTTCTCGGATGGATACCCATTAC CATCTTGGGACCGACG | 5' biotin |
| SEQ ID NO.336 | FLT3-NC-OP-15 | /biotin/CGTCGGTCCCTCAAGTGCTCGCAGAAGCATCGGCA AGTCAGGCGACCGACG | 5' biotin |
| SEQ ID NO.337 | FLT3-NC-OP-16 | /biotin/CGTCGGTCCCAGTGAGCTTATTTCACACGTTCTTTT CTATAGGGAGACCGACG | 5' biotin |
| SEQ ID NO.338 | IDH2-NC-OP-1 | /biotin/CGTCGGTCCATGGCGACCAGGTAGGCCAGGGTGG AGAGGGGATGACCGACG | 5' biotin |
| SEQ ID NO.339 | IDH2-NC-OP-2 | /biotin/CGTCGGTCTGGCTGGACCAAGCCCATCACCATTGG CAGGCACGGACCGACG | 5' biotin |
| SEQ ID NO.340 | IDH2-NC-OP-3 | /biotin/CGTCGGTCAGCCCATCATCTGCAAAAACATCCCAC GCCTAGTCGACCGACG | 5' biotin |
| SEQ ID NO.341 | IDH2-NC-OP-4 | /biotin/CGTCGGTCACTATCCGGAACATCCTGGGGGGGACT GTCTTCCGGACCGACG | 5' biotin |
| SEQ ID NO.342 | IDH2-NC-OP-5 | /biotin/CGTCGGTCGTTCAAGCTGAAGAAGATGTGGAAAA GTCCCAATGGACCGACG | 5' biotin |
| SEQ ID NO.343 | TP53-NC-OP-1 | /biotin/CGTCGGTCAGGGGGGAGCAGGGCTCACTCCAGGT GAGTGACCTGACCGACG | 5' biotin |
| SEQ ID NO.344 | TP53-NC-OP-2 | /biotin/CGTCGGTCCCTTGGAACTCAAGGATGCCCAGGCTG GGAAGGAGGACCGACG | 5' biotin |
| SEQ ID NO.345 | TP53-NC-OP-3 | /biotin/CGTCGGTCCGTGAGCGCTTCGAGATGTTCCGAGAG CTGAATGAGACCGACG | 5' biotin |
| SEQ ID NO.346 | TP53-NC-OP-4 | /biotin/CGTCGGTCTACTTCTCCCCCTCCTCTGTTGCTGCAG ATCCGTGGACCGACG | 5' biotin |
| SEQ ID NO.347 | TP53-NC-OP-5 | /biotin/CGTCGGTCGGGAGCCTCACCACGAGCTGCCCCCAG GGAGCACTGACCGACG | 5' biotin |
| SEQ ID NO.348 | TP53-NC-OP-6 | /biotin/CGTCGGTCGACCGGCGCACAGAGGAAGAGAATCT CCGCAAGAAGACCGACG | 5' biotin |
| SEQ ID NO.349 | TP53-NC-OP-7 | /biotin/CGTCGGTCCAGCTTTGAGGTGCGTGTTTGTGCCTG TCCTGGGAGACCGACG | 5' biotin |
| SEQ ID NO.350 | TP53-NC-OP-8 | /biotin/CGTCGGTCTTCCTATCCTGAGTAGTGGTAATCTACT GGGACGGGACCGACG | 5' biotin |
| SEQ ID NO.351 | TP53-NC-OP-9 | /biotin/CGTCGGTCCACCATCATCACACTGGAAGACTCCAG GTCAGGAGGACCGACG | 5' biotin |
| SEQ ID NO.352 | TP53-NC-OP-10 | /biotin/CGTCGGTCGTTCCTGCATGGGCGGCATGAACCGGA GGCCCATCGACCGACG | 5' biotin |
| SEQ ID NO.353 | TP53-NC-OP-11 | /biotin/CGTCGGTCGACTGTACCACCATCCACTACAACTAC ATGTGTAAGACCGACG | 5' biotin |
| SEQ ID NO.354 | TP53-NC-OP-12 | /biotin/CGTCGGTCCTCATCTTGGGCCTGTGTTATCTCCTAG GTTGGCTGACCGACG | 5' biotin |
| SEQ ID NO.355 | TP53-NC-OP-13 | /biotin/CGTCGGTCGTGTGGTGGTGCCCTATGAGCCGCCTG AGGTCTGGGACCGACG | 5' biotin |
| SEQ ID NO.356 | TP53-NC-OP-14 | /biotin/CGTCGGTCGTGTGGAGTATTTGGATGACAGAAACA CTTTTCGACAGACCGACG | 5' biotin |
| SEQ ID NO.357 | TP53-NC-OP-15 | /biotin/CGTCGGTCCCTCCTCAGCATCTTATCCGAGTGGAA GGAAATTTGACCGACG | 5' biotin |
| SEQ ID NO.358 | TP53-NC-OP-16 | /biotin/CGTCGGTCGCCTCTGATTCCTCACTGATTGCTCTTA GGTCTGGGACCGACG | 5' biotin |
| SEQ ID NO.359 | TP53-NC-OP-17 | /biotin/CGTCGGTCGCTCAGATAGCGATGGTGAGCAGCTGG GGCTGGAGGACCGACG | 5' biotin |
| SEQ ID NO.360 | TP53-NC-OP-18 | /biotin/CGTCGGTCACGGAGGTTGTGAGGCGCTGCCCCCAC CATGAGCGGACCGACG | 5' biotin |
| SEQ ID NO.361 | TP53-NC-OP-19 | /biotin/CGTCGGTCCCGCGCCATGGCCATCTACAAGCAGTC ACAGCACAGACCGACG | 5' biotin |
| SEQ ID NO.362 | TP53-NC-OP-20 | /biotin/CGTCGGTCTGTGGGTTGATTCCACACCCCCGCCCG GCACCCGCGACCGACG | 5' biotin |
| SEQ ID NO.363 | TP53-NC-OP-21 | /biotin/CGTCGGTCATGTTTTGCCAACTGGCCAAGACCTGC CCTGTGCAGACCGACG | 5' biotin |
| SEQ ID NO.364 | TP53-NC-OP-22 | /biotin/CGTCGGTCCTTCCTCTTCCTACAGTACTCCCCTGCC CTCAACAGACCGACG | 5' biotin |
| SEQ ID NO.365 | TP53-NC-OP-23 | /biotin/CGTCGGTCATCTGTTCACTTGTGCCCTGACTTTCAA CTCTGTCGACCGACG | 5' biotin |
| SEQ ID NO.366 | TP53-NC-OP-24 | /biotin/CGTCGGTCGGGCAGCTACGGTTTCCGTCTGGGCTT CTTGCATTGACCGACG | 5' biotin |
| SEQ ID NO.367 | TP53-NC-OP-25 | /biotin/CGTCGGTCCCCTGTCATCTTCTGTCCCTTCCCAGAA AACCTACGACCGACG | 5' biotin |
| SEQ ID NO.368 | TP53-NC-OP-26 | /biotin/CGTCGGTCCCTACACCGGCGGCCCCTGCACCAGCC CCCTCCTGGACCGACG | 5' biotin |
| SEQ ID NO.369 | TP53-NC-OP-27 | /biotin/CGTCGGTCAGAGGCTGCTCCCCCCGTGGCCCCTGC ACCAGCAGGACCGACG | 5' biotin |
| SEQ ID NO.370 | TP53-NC-OP-28 | /biotin/CGTCGGTCCTGAAGACCCAGGTCCAGATGAAGCTC CCAGAATGGACCGACG | 5' biotin |
| SEQ ID NO.371 | TP53-NC-OP-29 | /biotin/CGTCGGTCCCTATGGAAACTGTGAGTGGATCCATT GGAAGGGCGACCGACG | 5' biotin |
| SEQ ID NO.372 | TP53-NC-OP-30 | /biotin/CGTCGGTCGCGTCGAGCCCCCTCTGAGTCAGGAAA CATTTTCAGACCGACG | 5' biotin |
| SEQ ID NO.373 | TP53-NC-OP-31 | /biotin/CGTCGGTCCGGGTCACTGCCATGGAGGAGCCGCA GTCAGATCCGACCGACG | 5' biotin |
| SEQ ID NO.374 | TP53-NC-OP-32 | /biotin/CGTCGGTCTCCCCACTTTTCCTCTTGCAGCAGCCAG ACTGCCTGACCGACG | 5' biotin |
| SEQ ID NO.375 | IDH1-NC-OP-1 | /biotin/CGTCGGTCGCTTATGGGGATCAAGTAAGTCATGTT GGCAATAATGGACCGACG | 5' biotin |
| SEQ ID NO. 376 | IDH1-NC-OP-2 | /biotin/CGTCGGTCGAGTGGATGGGTAAAACCTATCATCAT AGGTCGTCGACCGACG | 5' biotin |
| SEQ ID NO. 377 | IDH1-NC-OP-3 | /biotin/CGTCGGTCGAGAAGCCATTATCTGCAAAAATATCC CCCGGCTTGACCGACG | 5' biotin |
| SEQ ID NO.378 | IDH1-NC-OP-4 | /biotin/CGTCGGTCGGCACCATACGAAATATTCTGGGTGGC ACGGTCTTGACCGACG | 5' biotin |
| SEQ ID NO.379 | IDH1-NC-OP-5 | /biotin/CGTCGGTCGTTGAGGAGTTCAAGTTGAAACAAATG TGGAAATCACCAAGACCGACG | 5' biotin |

After testing the basic effect of the NC probes of the present invention, Examples 4-8 further test the hybrid capture system based on the NC probes of the present invention and related parameters.

### Example 4: Optimal NC probe concentration test

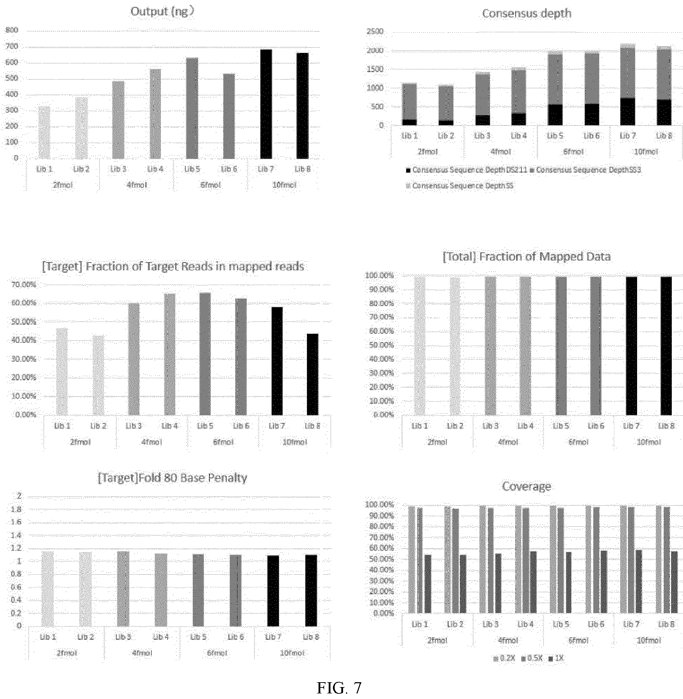

The difference in capture efficiency of NC probes with different concentrations for target genes is unknown, and through an experiment in which probes of different concentration gradients are set, the optimal probe concentration is sought. A specific experimental protocol is shown in Table 9 below, and a target region of 4.5 kb is designed according to the probe design concept of the present invention, and a Promega standard male (G1471 Promega-male) is used to fragment a sample to about 200-250 bp. For a specific experimental process, other variables are consistent except for different probe concentrations in experimental groups. Result data is shown in FIG. 7.

**Table 9**

| **Experimental group** | **Probe concentration** |
|---|---|
| Lib 1 | 2 fmol |
| Lib 2 | 2 fmol |
| Lib 3 | 4 fmol |
| Lib 4 | 4 fmol |
| Lib 5 | 6 fmol |
| Lib 6 | 6 fmol |
| Lib 7 | 10 fmol |
| Lib 8 | 10 fmol |

From result analysis of the Consensus depth, DS211 or SS information is directly proportional to the NC probe concentration. When the NC probe concentration is lower, less effective library information is captured, the higher the NC probe concentration, the richer the captured effective library information, but a too high NC probe concentration will lead to an excess of redundant NC probes in the system, resulting in a decrease in on-target rate. The optimal NC probe concentration used in this system is 6-10 fmol, with 6 fmol of the NC probe being more preferred.

### Example 5: Optimal hybrid capture temperature test

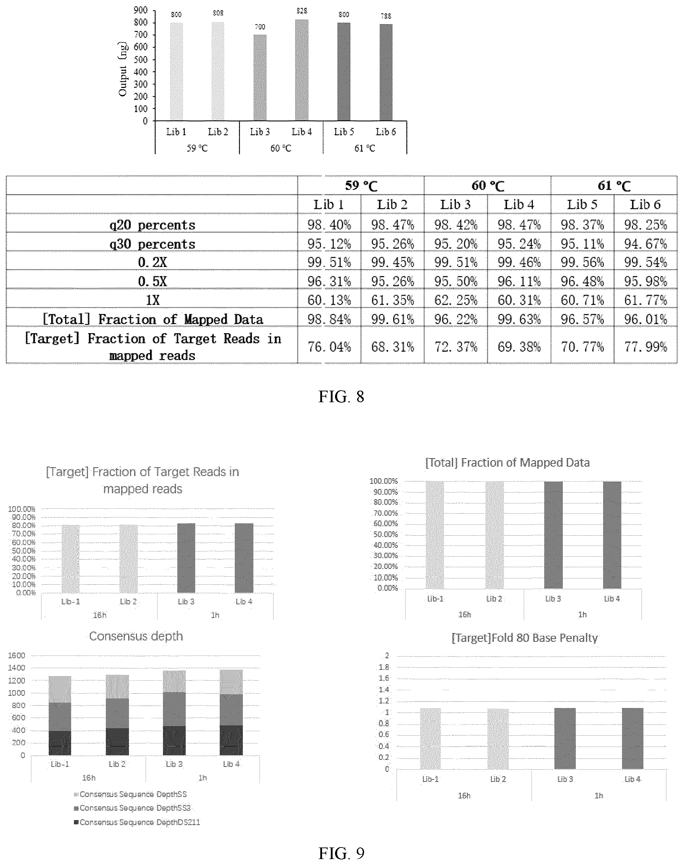

This system uses the NC probes, and the hybrid capture temperature needs to be selected according to the probe structure. In order to determine the optimal temperature conditions, a series of tests are performed. A specific experimental protocol is shown in Table 10 below. A target region of 4.5 kb is designed according to the design concept of the NC probes of the present invention. A Promega standard male is used to fragment a sample to about 200-250 bp. For a specific experimental process, other variables are consistent except for different hybrid capture temperatures in experimental groups. Result data is shown in FIG. 8.

**Table 10**

| **Experimental group** | **Hybrid capture temperature** |
|---|---|
| Lib 1 | 57°C |
| Lib 2 | 60°C |
| Lib 3 | 63°C |

From result analysis of the library construction efficiency and the Consensus depth, the DS211 or SS content is affected by the hybrid capture temperature, the hybrid capture temperature of 60°C performs better than the other two temperature conditions, and the capture efficiency and the on-target rate at the hybrid capture temperature of 60°C are higher than those at the other hybrid capture temperatures.

To ensure that 60°C is the optimal hybridization condition, and that this system is not too sensitive to the hybridization temperature, closer hybridization conditions are then tested to compare the difference in library capture efficiency under hybridization conditions of 59°C, 60°C, and 61°C (see Table 11), other variables are consistent except for different hybrid capture temperatures in experimental groups, and the result data is shown in FIG. 8.

**Table 11**

| **Experimental group** | **Hybrid capture temperature** |
|---|---|
| Lib 1 | 59°C |
| Lib 2 | 60°C |
| Lib 3 | 61°C |

From the above data analysis, hybridization temperatures from 59°C to 61°C show a superior capture efficiency, with 60°C being used as the final hybrid capture condition for this system.

### Example 6: Shortening hybrid capture time

The hybridization time used in a traditional hybrid capture system is 16 hours, while the hybridization time used in the present invention can be reduced from 16 hours to 1 hour, and shortening the hybridization time does not affect the efficiency of the probes in capturing DNA samples.

An experiment is carried out by using the hybrid capture conditions of this system, a specific experimental protocol is shown in Table 12 below, a target region of 50 kb is first designed according to the design idea of the NC probes of the present invention, and a GW-OGTM800 standard is used to fragment a sample to about 200-250 bp.

The experimental process is as follows:
gDNA is fragmented to about 200 bp (Covaris ultrasonic fragmentation instrument), and end repair and adapter ligation are carried out, followed by purification of nucleic acid using an equal volume of Beads; and this specific purification process is as follows:
1. NadPrep^{®} SP Beads are taken out in advance, vortexing is conducted for uniform mixing, and equilibration is conducted at room temperature for 30 minutes before use;
2. 80µL of NadPrep^{®} SP Beads is added to the adapter ligation product to be uniformly mixed, and the mixture is incubated at 25°C for 5-10 minutes;
3. a PCR tube is instantaneously centrifuged, and placed on a magnetic rack for 5-10 minutes until liquid is completely clear, and a supernatant is discarded by pipetting with a pipette;
4. 200µL of BW Buffer is added for washing once, the washed material is allowed to stand for 2 minutes, and a supernatant is discarded by pipetting; and
5. a hybridization reaction solution is added to the reaction system.

A hybridization system contains 6 fmol of probe, 1×Hyb Buffer, 1×Enhance, 1µg of Human Cot-1, and 100 pmmol of Blocker, and the configured hybridization reaction system is placed in a temperature controller for a reaction under the following conditions: denaturation at 95°C for 2 minutes, and hybridization at 60°C for 1 hour or 16 hours.

After completion of the hybridization reaction, the supernatant is transferred to a new PCR tube, and 10µL of M270 Beads is added to the PCR reaction tube for hybrid capture at 60°C for 20 minutes.

After the end of 20 minutes of capture, washing is separately performed once with an elution buffer I, an elution buffer II, and an elution buffer III.

After washing is completed, a PCR reaction system is added to the M270 Beads, wherein the PCR reaction system mainly includes 2×HiFi PCR Master Mix, 5µL of Index Primer Mix, and 20µL of TE; a PCR amplification procedure is started on a PCR temperature controller, and after the reaction is finished, the resulting product is purified by using 1×magnetic beads, and the purified product is sequenced on an Illumina^{®} platform. Test result data is shown in FIG. 9.

**Table 12**

| **Experimental group** | **Library construction and hybrid capture kit** | **Hybridization time** |
|---|---|---|
| Lib 1 | EASY Hybrid Capture System | 16 hours |
| Lib 2 | EASY Hybrid Capture System | 16 hours |
| Lib 3 | EASY Hybrid Capture System | 1 hour |
| Lib 4 | EASY Hybrid Capture System | 1 hour |

From result analysis of the Consensus depth, DS211 or SS information is directly proportional to the hybridization time, 90% or more of the efficient library have been captured after 1 hour of hybridization, with the final selection of the hybridization time of 1 hour, and the entire experimental process is controlled to be completed in one day.

**Example 7:** Comparison of capture of a small target region by the NC probes in a PCR-free mode with a conventional capture process with conventional probes

In order to compare the performance of capture with NC probes in an optimized PCR-free mode with that of capture with traditional probes in a non-PCR-free mode for a small target region, an experiment is performed according to a grouping method in Table 13 below, wherein a group 1 uses a traditional manner to construct a targeted capture library, with the traditional hybrid capture system matched with probes of 120 nt; and a group 2 uses a system of the NC probes of the present invention to construct a PCR-free targeted capture library, capture probes are designed for a same region, the probes cover genomic exon regions, and the target region size is about 4 kb.

**Table 13**

| **Experimental group** | **Library construction kit** | **Hybrid capture kit** | |
|---|---|---|---|
| Group 1 | NadPrep DNA universal library construction kit | NadPrep^{®} Hybrid Capture Reagents | Control group |
| Group 2 | EASY Hybrid Capture System | EASY Hybrid Capture System | Experimental group |

Wherein a specific implementation process in the group 1 refers to a commercial instruction for a NadPrep^{®} simple hybrid capture kit; while a specific experimental process in the group 2 refers to that in Example 6, and the hybridization time is fixed at 1 hour.

The data performance of this example is shown in Table 14. The mean coverage in the group 1 and the group 2 is close to 100%, while the on-target rate in the group 2 is 59%, which is higher than 11.73% in the group 1. It is obvious that the system of the NC probes of the present invention can effectively improve the on-target rate.

**Table 14. Small target region capture efficiency higher than traditional hybrid capture**

| | **Group 1 Traditional** | **Group 2 EASY Cap** |
|---|---|---|
| Fraction of Target Reads in mapped reads | 11.73% | 59% |
| Fraction of Mapped Reads | 99.29% | 99.32% |
| 0.2×Mean coverage | 100.00% | 100% |
| 0.5×Mean coverage | 98.92% | 100% |
| Fold 80 base penalty | 1.17 | 1.12 |
| Note: | | |
| •[Target] Fraction of Target Reads in mapped reads: a proportion of target reads in mapped reads. | | |
| •Fraction of Mapped reads: a proportion of reads mapped to a human genome in all reads. | | |
| •0.2×Mean Coverage: 0.2×mean coverage percentage. | | |
| •0.5×Mean coverage: 0.5×mean coverage percentage. | | |
| •Fold 80 Base Penalty: sequencing multiples required to be increased to ensure that 80% of target bases reaches the original average coverage depth. | | |

### Example 8: Detection efficiency of fusion genes higher than traditional hybrid capture

Fusion genes are produced when partial fragments of two genes are joined due to genome rearrangement. The fusion genes can be detected and analyzed by capturing and sequencing regions on both sides of a rearrangement breakpoint. Due to the fact that only part of rearrangement fragments across the breakpoint is the original sequence, for conventional probes, there may be a problem where only part of the fragments can be bound. The NC probes can also improve the detection ability of fusion genes through more probe binding possibilities.

**An** experiment is carried out according to a grouping method Table 15 below, wherein Group 1 uses a conventional manner to construct a targeted capture library, with a traditional hybrid capture system matched with probes of 120nt, and probes covering the ROS1 intron 33 are designed to detect CD74-ROS1 fusion; and Group 2 uses the present invention to construct a targeted capture library, with capture probes designed for the same region, a target region of about 1 kb. Wherein a specific implementation process in the group 1 refers to the commercial instruction for the NadPrep^{®} simple hybrid capture kit.

**Table 15**

| **Experimental group** | **Library construction kit** | **Hybrid capture kit** | |
|---|---|---|---|
| Group 1 | NadPrep DNA universal library construction kit | NadPrep^{®} Hybrid Capture Reagents | Control group |
| Group 2 | EASY Hybrid Capture System | EASY Hybrid Capture System | Experimental group |

The sample is a pan-tumor 800 gDNA standard (GW-OGTM800) containing multiple digital PCR verified mutation sites, one of which is CD74-ROS1 Fusion, and this site has a theoretical mutation frequency of 6%.

The specific experimental process in the group 2 refers to that in Example 6, and result data is shown in Table 16 below.

**Table 16 Detection efficiency of fusion genes higher than traditional hybrid capture**

| | **Group 1 Traditional** | **Group 2 EASY Cap** |
|---|---|---|
| Fraction of Target Reads in mapped reads | 10.5% | 52.3% |
| Fraction of Mapped Reads | 98.08% | 99.88% |
| 0.2×Mean coverage | 98.46% | 100.00% |
| 0.5×Mean coverage | 89.57% | 88.24% |
| CD74-ROS1 (a theoretical value of 6%) | 1.1% | 5.8% |
| Note: | | |
| •[Target] Fraction of Target Reads in mapped reads: a proportion of target reads in mapped reads. | | |
| •Fraction of Mapped reads: a proportion of reads mapped to a human genome in all reads. | | |
| •0.2×Mean Coverage: 0.2×mean coverage percentage. | | |
| •0.5×Mean coverage: 0.5×mean coverage percentage. | | |
| •Fold 80 Base Penalty: sequencing multiples required to be increased to ensure that 80% of target bases reaches the original average coverage depth. | | |

Fusion sites are often located within a repeating region, and a probe design within the repeating region is something of a capture challenge. However, the use of the NC probes in this system shows certain advantages for the detection of the fusion genes. The GW-OGTM800 standard in this experiment contains a set of CD74-ROS1 fusion genes with a mutation frequency of 5% as verified by digital PCR; and the Group 1 and the Group 2 use probes covering the same region for hybrid capture, and the frequency of detecting fusion genes by the traditional method is about 1.1%, while the frequency of detecting fusion genes by the optimized system of the present invention is 5.8%.

## Claims

1. A liquid-phase hybrid capture method, comprising the following steps of:
(1) probe design:
designing a pool of probes based on nucleic acid target regions, wherein each probe comprises a respective target specific sequence complementarily pairing with a respective nucleic acid target sequence, and each probe comprises a same probe binding sequence, the probe binding sequence comprises a first probe binding sequence and a second probe binding sequence, wherein a 5' end of each probe has the first probe binding sequence complementarily pairing with a 3' end of another probe, and a 3' end of each probe has the second probe binding sequence complementarily pairing with a 5' end of another probe, and ,
synthesizing each oligonucleotide based on corresponding sequence, and
modifying a 5' or 3' end with a biomarker;
(2) library construction;
(3) hybrid capture
i. configuring a hybridization system: wherein the hybridization system comprises the probes; and
ii. carrying out a hybridization reaction: placing the hybridization system at 57-63°C for hybridization for 1-2h;
(4) product capture: after the hybridization reaction is completed, adding streptavidin magnetic beads to the reaction system for hybrid capture;
(5) product elution: after capture is completed, washing the captured product once with a first elution buffer, a second elution buffer and a third elution buffer sequentially; and
(6) product amplification and purification: after the washing is completed, adding a PCR reaction system for a PCR amplification procedure, and after the reaction is completed, performing purification by using magnetic beads.

2. The method according to claim 1, wherein the nucleic acid is from fresh tissue, frozen tissue, paraffin embedded tissue, hydrothorax and ascites, plasma or exfoliated tumor cell tissue.

3. The method according to claim 1, wherein the nucleic acid is plasma free DNA, genomic DNA or RNA.

4. The method according to claim 1, wherein the library construction is to construct a DNA library based on nucleic acid fragment size of 200-250 bp.

5. The method according to claim 1, wherein the hybridization system comprises 2-10 fmol of the probe, 1×Hyb Buffer, 1×Enhance, 1µg of Human Cot-1, and 100 pmmol of Blocker.

6. The method according to claim 5, wherein the hybridization system comprises 6 fmol of the probe.

7. The method according to claim 1, wherein the PCR reaction system comprises 2×HiFi PCR Master Mix, 5 µL of Index Primer Mix and 20 µL of TE.

8. The method according to claim 1, wherein the library construction comprises end repair and adapter ligation of nucleic acid fragments.

9. The method according to claim 1, wherein formula of the first elution buffer is 5×SSPE, and 0.5-5% of SDS; formula of the second elution buffer is 2×SSPE, and 0.05-0.5% of SDS; and formula of the third elution buffer is 0.1×SSPE, and 0.005%-0.05% of SDS.

10. A liquid-phase hybrid capture kit, comprising the following components: probes, a hybridization reaction solution, an elution buffer, and nucleic acid purification magnetic beads; wherein the probes are designed based on nucleic acid target regions, wherein each probe comprises a respective target specific sequence complementarily pairing with a respective nucleic acid target sequence, and each probe comprises a same probe binding sequence, the probe binding sequence comprises a first probe binding sequence and a second probe binding sequence, wherein a 5' end of each probe has the first probe binding sequence complementarily pairing with a 3' end of another probe, and a 3' end of each probe has the second probe binding sequence complementarily pairing with a 5' end of another probe.

11. The kit according to claim 10, further comprising an end repair enzyme mixture, an end repair reaction buffer, a molecular tag-containing adapter, library amplification primers, a PCR premix, an adapter blocker, a DNA blocker, a hybridization enhancer, a magnetic bead wash buffer, and capture library PCR primers.

12. Use of the method according to any one of claims 1-9 in genomic target region capture.

13. The use according to claim 12, wherein the target region capture is used for low-frequency mutation detection, chromosome copy number variation analysis, and insertion/deletion, microsatellite instability or fusion gene detection in nucleic acid fragments; or is used for targeted metagenomic next-generation sequencing (mNGS), and epidemiological detection of pathogens.

## Patentansprüche

1. Hybrides Flüssigphasenerfassungsverfahren, umfassend die folgenden Schritte:
(1) Sondengestaltung:
Gestalten eines Pools von Sonden basierend auf Nukleinsäurezielregionen, wobei jede Sonde eine jeweilige zielspezifische Sequenz umfasst, die sich komplementär mit einer jeweiligen Nukleinsäurezielsequenz paart, und jede Sonde eine gleiche Sondenbindungssequenz umfasst, die Sondenbindungssequenz eine erste Sondenbindungssequenz und eine zweite Sondenbindungssequenz umfasst, wobei ein 5'-Ende jeder Sonde die erste Sondenbindungssequenz aufweist, die sich komplementär mit einem 3'-Ende einer anderen Sonde paart, und ein 3'-Ende jeder Sonde die zweite Sondenbindungssequenz aufweist, die sich komplementär mit einem 5'-Ende einer anderen Sonde paart, und
Synthetisieren jedes Oligonukleotids basierend auf entsprechender Sequenz und
Modifizieren eines 5'- oder 3'-Endes mit einem Biomarker;
(2) Bibliothekskonstruktion;
(3) Hybriderfassung
i. Konfigurieren eines Hybridisierungssystems: wobei das Hybridisierungssystem die Sonden umfasst; und
ii. Durchführung einer Hybridisierungsreaktion: Platzieren des Hybridisierungssystems bei 57-63 °C für Hybridisierung für 1-2 h;
(4) Produkterfassung: nachdem die Hybridisierungsreaktion abgeschlossen ist, Hinzufügen von Streptavidin-Magnetkügelchen zu dem Reaktionssystem für Hybriderfassung;
(5) Produktelution: nachdem Erfassung abgeschlossen ist, Waschen des erfassten Produkts einmal nacheinander mit einem ersten Elutionspuffer, einem zweiten Elutionspuffer und einem dritten Elutionspuffer; und
(6) Produktamplifikation und -reinigung: nachdem das Waschen abgeschlossen ist, Hinzufügen eines PCR-Reaktionssystems für einen PCR-Amplifikationsvorgang und nachdem die Reaktion abgeschlossen ist, Ausführen von Reinigung durch Verwenden von Magnetkügelchen.

2. Verfahren nach Anspruch 1, wobei die Nukleinsäure aus frischem Gewebe, gefrorenem Gewebe, in Paraffin eingebettetem Gewebe, Hydrothorax und Aszites, Plasma oder exfoliertem Tumorzellgewebe ist.

3. Verfahren nach Anspruch 1, wobei die Nukleinsäure plasmafreie DNA, genomische DNA oder RNA ist.

4. Verfahren nach Anspruch 1, wobei die Bibliothekskonstruktion eine DNA-Bibliothek basierend auf Nukleinsäurefragmentgröße von 200-250 bp konstruieren soll.

5. Verfahren nach Anspruch 1, wobei das Hybridisierungssystem 2-10 fmol der Sonde, 1×Hyb-Puffer, 1×Enhance, 1 µg Human-Cot-1 und 100 pmmol Blocker umfasst.

6. Verfahren nach Anspruch 5, wobei das Hybridisierungssystem 6 fmol der Sonde umfasst.

7. Verfahren nach Anspruch 1, wobei das PCR-Reaktionssystem 2×HiFi-PCR-Master-Mix, 5 µl Index-Primer-Mix und 20 µl TE umfasst.

8. Verfahren nach Anspruch 1, wobei die Bibliothekskonstruktion Endreparatur und Adapterligierung von Nukleinsäurefragmenten umfasst.

9. Verfahren nach Anspruch 1, wobei Formel des ersten Elutionspuffers 5×SSPE und 0,5-5 % von SDS ist; Formel des zweiten Elutionspuffers 2×SSPE und 0,05-0,5 % von SDS ist; und Formel des dritten Elutionspuffers 0,1×SSPE und 0,005 %-0,05 % von SDS ist.

10. Hybrides Flüssigphasenerfassungskit, umfassend die folgenden Komponenten: Sonden, eine Hybridisierungsreaktionslösung, einen Elutionspuffer und Nukleinsäurereinigungsmagnetkügelchen; wobei die Sonden basierend auf Nukleinsäurezielregionen gestaltet sind, wobei jede Sonde eine jeweilige zielspezifische Sequenz umfasst, die sich komplementär mit einer jeweiligen Nukleinsäurezielsequenz paart, und jede Sonde eine gleiche Sondenbindungssequenz umfasst, die Sondenbindungssequenz eine erste Sondenbindungssequenz und eine zweite Sondenbindungssequenz umfasst, wobei ein 5'-Ende jeder Sonde die erste Sondenbindungssequenz aufweist, die sich komplementär mit einem 3'-Ende einer anderen Sonde paart, und ein 3'-Ende jeder Sonde die zweite Sondenbindungssequenz aufweist, die sich komplementär mit einem 5'-Ende einer anderen Sonde paart.

11. Kit nach Anspruch 10, ferner umfassend eine Endreparaturenzymmischung, einen Endreparaturreaktionspuffer, einen ein molekulares Tag enthaltenden Adapter, Bibliotheksamplifikationsprimer, einen PCR-Prämix, einen Adapterblocker, einen DNA-Blocker, einen Hybridisierungsverstärker, einen Magnetkügelchenwaschpuffer und Erfassungsbibliotheks-PCR-Primer.

12. Verwendung des Verfahrens nach einem der Ansprüche 1-9 bei genomischer Zielregionerfassung.

13. Verwendung nach Anspruch 12, wobei die Zielregionerfassung für Niederfrequenzmutationsdetektion, Chromosomenkopienanzahlvariationsanalyse und Insertion/Deletion, Mikrosatelliteninstabilität oder Fusionsgendetektion in Nukleinsäurefragmenten verwendet wird; oder für gezieltes metagenomisches Sequenzieren der nächsten Generation (mNGS) und epidemiologische Detektion von Pathogenen verwendet wird.

## Revendications

1. Procédé de capture hybride en phase liquide, comprenant les étapes suivantes de :
(1) conception de sonde :
conception d'un ensemble de sondes sur la base de régions cibles d'acide nucléique, où chaque sonde comprend une séquence spécifique cible respective s'appariant de manière complémentaire à une séquence cible d'acide nucléique respective, et chaque sonde comprend une même séquence de liaison de sonde, la séquence de liaison de sonde comprend une première séquence de liaison de sonde et une seconde séquence de liaison de sonde, où une extrémité 5' de chaque sonde comporte la première séquence de liaison de sonde s'appariant de manière complémentaire à une extrémité 3' d'une autre sonde, et une extrémité 3' de chaque sonde comporte la seconde séquence de liaison de sonde s'appariant de manière complémentaire à une extrémité 5' d'une autre sonde, et,
synthèse de chaque oligonucléotide sur la base d'une séquence correspondante, et
modification d'une extrémité 5' ou 3' avec un biomarqueur ;
(2) construction de banque ;
(3) capture hybride
i. configuration d'un système d'hybridation : ledit système d'hybridation comprenant les sondes ; et
ii. réalisation d'une réaction d'hybridation : mise en place du système d'hybridation à 57 à 63 °C pour hybridation pendant 1 à 2 h ;
(4) capture de produit : une fois la réaction d'hybridation terminée, ajout de billes magnétiques de streptavidine au système de réaction pour la capture hybride ;
(5) élution de produit : une fois la capture terminée, lavage du produit capturé une fois avec un premier tampon d'élution, un deuxième tampon d'élution et un troisième tampon d'élution séquentiellement ; et
(6) amplification et purification de produit : une fois le lavage terminé, ajout d'un système de réaction PCR pour une procédure d'amplification PCR, et une fois la réaction terminée, réalisation d'une purification à l'aide de billes magnétiques.

2. Procédé selon la revendication 1, dans lequel l'acide nucléique provient de tissu frais, de tissu congelé, de tissu inclus en paraffine, d'hydrothorax et d'ascite, de plasma ou de tissu de cellules tumorales exfoliées.

3. Procédé selon la revendication 1, dans lequel l'acide nucléique est un ADN plasmatique

4. Procédé selon la revendication 1, dans lequel la construction de banque consiste à construire une banque d'ADN sur la base d'une taille de fragment d'acide nucléique de 200 à 250 pb.

5. Procédé selon la revendication 1, dans lequel le système d'hybridation comprend 2 à 10 fmol de la sonde, un tampon d'hybridation 1×, un réactif d'amélioration 1×, 1 µg de Cot-1 humain et 100 pmmol de bloqueur.

6. Procédé selon la revendication 5, dans lequel le système d'hybridation comprend 6 fmol de la sonde.

7. Procédé selon la revendication 1, dans lequel le système de réaction PCR comprend un Master Mix PCR HiFi 2×, 5 µL de mélange d'amorce d'index et 20 µL de TE.

8. Procédé selon la revendication 1, dans lequel la construction de banque comprend une réparation d'extrémité et une ligature d'adaptateur de fragments d'acide nucléique.

9. Procédé selon la revendication 1, dans lequel la formule du premier tampon d'élution est 5×SSPE, et 0,5 à 5 % de SDS ; la formule du deuxième tampon d'élution est 2×SSPE, et 0,05 à 0,5 % de SDS ; et la formule du troisième tampon d'élution est 0,1×SSPE, et 0,005 % à 0,05 % de SDS.

10. Kit de capture hybride en phase liquide, comprenant les composants suivants : des sondes, une solution de réaction d'hybridation, un tampon d'élution et des billes magnétiques de purification d'acide nucléique ; dans lequel les sondes sont conçues sur la base de régions cibles d'acide nucléique, dans lequel chaque sonde comprend une séquence spécifique cible respective s'appariant de manière complémentaire à une séquence cible d'acide nucléique respective, et chaque sonde comprend une même séquence de liaison de sonde, la séquence de liaison de sonde comprend une première séquence de liaison de sonde et une seconde séquence de liaison de sonde, dans lequel une extrémité 5' de chaque sonde comporte la première séquence de liaison de sonde s'appariant de manière complémentaire à une extrémité 3' d'une autre sonde, et une extrémité 3' de chaque sonde comporte la seconde séquence de liaison de sonde s'appariant de manière complémentaire à une extrémité 5' d'une autre sonde.

11. Kit selon la revendication 10, comprenant en outre un mélange enzymatique de réparation d'extrémité, un tampon de réaction de réparation d'extrémité, un adaptateur contenant une étiquette moléculaire, des amorces d'amplification de banque, un prémélange PCR, un bloqueur d'adaptateur, un bloqueur d'ADN, un réactif d'amélioration d'hybridation, un tampon de lavage à billes magnétiques et des amorces PCR de banque de capture.

12. Utilisation du procédé selon l'une quelconque des revendications 1 à 9 dans la capture de région cible génomique.

13. Utilisation selon la revendication 12, dans laquelle la capture de région cible est utilisée pour la détection de mutations à faible fréquence, l'analyse de la variation du nombre de copies chromosomiques et l'insertion/délétion, l'instabilité de microsatellites ou la détection de gènes de fusion dans des fragments d'acide nucléique ; ou est utilisée pour le séquençage ciblé métagénomique de nouvelle génération (mNGS) et la détection épidémiologique d'agents pathogènes.
